# EUROPÄISCHE PATENTSCHRIFT

(11) **EP 1 430 349 B1**
(45) Veröffentlichungstag und Bekanntmachung des Hinweises auf die Patenterteilung: **09.12.2009**
(21) Anmeldenummer: 00971341.3
(22) Anmeldetag: 07.10.2000
(51) Int. Cl.: G02B 27/01, A61B 3/12

(54) **INFORMATIONSSYSTEM**
INFORMATION SYSTEM
SYSTEME D'INFORMATION

(43) Veröffentlichungstag der Anmeldung: 23.06.2004
(73) Patentinhaber: Metaio Gmbh, 80797 München (DE)
(72) Erfinder: EBERL, Heinrich, A., 87463 Probstried (DE); EBERL, Roland, H., C., 80687 München (DE); DICKERSON, David, P., 85354 Freising (DE); KÖNIGSTEIN, Karsten, 82229 Seefeld (DE)
(74) Vertreter: Dickerson, David
(86) Internationale Anmeldenummer: PCT/EP2000/009843
(87) Internationale Veröffentlichungsnummer: WO 2002/031580

(56) Entgegenhaltungen:
- DE-A- 19 631 414
- DE-A- 19 728 890
- US-A- 5 815 741

## Beschreibung

### Gebiet der Erfindung

Die vorliegende Offenbarung betrifft ein Informationssystem auf der Basis einer Gesichtsfelderfassungsvorrichtung, die sichtbares Licht aus dem natürlich wahrgenommenen Gesichtsfeld eines Auges erfaßt, und ein entsprechendes Verfahren zum Zurverfügungstellen von Informationen.

### Verwandte Anmeldungen

Aus den deutschen Offenlegungsschriften DE 196 31 414 A1 und DE 197 28 890 sind optische Vorrichtungen bekannt, die eine Aufnahme des Netzhautreflexbildes und eine Überlagerung von Zusatzbildern im Auge ermöglichen. In zwei deutschen Patentanmeldungen, deren Einreichungstag und Anmelder mit dem dieser Anmeldung übereinstimmen, werden sowohl Weitergestaltungen dieser optischen Vorrichtungen als auch Systeme beschreiben, die diese Vorrichtungen ergänzen oder gar ersetzen. Insbesondere werden darin die Justierung der obengenannten optischen Vorrichtung sowie ein System beschrieben, das auf neue Art und Weise auch in der Lage ist, Bilder auf ein Auge zu Projizieren. Letzteres System basiert auf einer Projektion eines Bildes auf die Netzhaut des Auges, die gleichzeitig, jedoch nacheilend zu einer scanartigen Erfassung des Netzhautreflexbildes erfolgt.

Da die in den obengenannten Anmeldungen beschriebenen Vorrichtungen und Systeme bevorzugt in Form einer Brille ausgestaltet sind, werden sie nachfolgend der Einfachheit halber auch als Brillensystem bezeichnet. Diese Bezeichnung impliziert keine Einschränkung. Selbstverständlich sind auch andere Ausführungsformen solcher Vorrichtungen und Systeme in den unten beschriebenen Zusammenhängen anstelle des "Brillensystems" gleichfalls anwendbar.

In der DE 196 31 414 A1 werden zahlreichen Anwendungsmöglichkeiten des darin offenbarten Brillensystems angesprochen, ohne daß diese genauer beschrieben werden konnten. Ein Miterfinder des ursprünglichen Brillensystems hat jetzt in Zusammenarbeit mit einem Forschungsteam die Anwendungsmöglichkeiten der oben erwähnten Brillensysteme genauer untersucht . Aus Überlegungen der wirtschaftlichen Implementierbarkeit sind Weiterbildungen und Abänderungen der bisher offenbarten Brillensysteme entstanden, die im Rahmen dieser und zwei weiterer Patentanmeldungen desselben Einreichungstags und desselben Anmelders angemeldet werden. Zu den vom Forscherteam entwickelten Systemmerkmale, die in vielen der beschriebenen Ausführungsformen wiederkehren, gehören, daß das System:
- ein Hornhautreflexbild des Auge mindest teilweise erfaßt;
- ein Teil des auf das Auge einfallenden Lichtes mittels einer sphärischen oder sphärisch wirkenden Reflektionsschicht in eine Sensorvorrichtung lenkt;
- das Netzhautbild über den Oxidierungsgrad der Netzhautzäpfen und/oder der Netzhautstäbchen ermittelt;
- lediglich eine partielle Erfassung eines Netzhautreflexbildes vornimmt; und/oder
- eine Gesichtsfelderfassungsvorrichtung umfaßt, die sichtbares Licht aus dem natürlich wahrgenommenen Gesichtsfeld erfaßt, ohne ein Netzhautreflexbild zu erfassen.

Zwecks Verfahrensökonomie wurde der Inhalt dieser drei Patentanmeldungen nicht nach diesen Systemmerkmalen aufgeteilt. Stattdessen betrifft jede dieser drei Anmeldungen ein jeweiliges, zusammenfassendes Grundkonzept. Diese sind:
- die Ausführung des Brillensystems als Informationssystem, das Informationen in Abhängigkeit von einem natürlich wahrgenommenen Gesichtsfeld eines Menschen zur Verfügung stellt;
- die Ausführung des Brillensystems als Informationssystem, das Informationen in Abhängigkeit von aus einem Auge erfaßten Signale zur Verfügung stellt, diese jedoch nicht in das Auge projiziert, aus dem die Signale erfaßt worden sind; und
- die Ausführung des Brillensystems als Informationssystem, das Informationen in Abhängigkeit von aus einem Auge erfaßten Signale zur Verfügung stellt, wobei die Informationen zumindest teilweise in das Auge projiziert, die Signale jedoch nicht in der aus der DE 196 31 414 A1 bekannten Weise erfaßt werden.

Es sei zu erwähnen, daß viele Anwendungen der konzipierten Brillensysteme auf einer Kombination mehrerer der obengenannten Grundkonzepte basieren, wodurch eine natürliche Verflechtung der zugehörigen drei Anmeldungen entsteht. Dementsprechend besteht eine gewisse Redundanz zwischen diesen drei Anmeldungen. Jedoch wird bei Detailfragen, die sich primär mit nur einem der Systemkonzepte beschäftigen, hiermit explizit quer auf die dieses Grundkonzept betreffende Anmeldung verwiesen.

### Stand der Technik /. Technischer Hintergrund

Es sind viele Fälle aus dem Alltag bekannt, bei dem es nützlich und/oder wünschenswert wäre, sofort über Informationen zu verfügen, die über unser persönliches Wissen und Sinnesempfindungen hinausgehen. Beispiele hierfür sind das Suchen einer Unterputz-Elektroleitung in einer Wand, die Navigation in einer fremden Stadt, das Sammeln von Wildpilzen und das Untersuchen eines möglicherweise gefährlichen Objekts mittels eines ferngesteuerten Roboters.

Die starke Abhängigkeit von sehenden Menschen an ihre Sehempfindungen trägt deutlich dazu bei, daß zusätzliche Informationen nur schwer zur Verfügung gestellt werden können. Denn die Tatsache, daß sehende Menschen vorwiegend mit den Augen wahrnehmen, macht es in vielen Fällen erforderlich, daß die Zusatzinformationen entweder über die Augen zugespeist oder anhand der gesehenen Informationen ermittelt werden. Bei einer Zuspeisung über die Augen muß jedoch die Ausrichtung der Augen genaustens berücksichtigt werden, um ein richtiges "Plazieren" und ein "Verwackeln" oder "Verwischen" der zugespeisten Informationen zu vermeiden. Zudem sollen die Informationen in vielen Fällen ohne gezielte Bewegung der Augenäpfel zugänglich gemacht werden; ein Autofahrer mag zwar eine Landkarte auf seinem Schoß haben, möchte aber ungern von der Straße wegschauen müssen.

Durch ihre Bindung an feste Medien, z.B. Papier, CRT- und LCD-Bildschirme, u.s.w., sind bisherige visuelle Informationssysteme nicht in der Lage gewesen, die Komfortbedürfnisse eines sehenden Menschen ausreichend nachzukommen. Nicht visuellen Informationssystemen fehlte bisher die für sehende Menschen selbstverständliche Kopplung an das Gesehene.

Näheres zum Stand der Technik enthält die DE 196 31 414 A1, deren Einleitung mehrere moderne Informationssysteme, insbesondere aus dem Militärbereich, beschreibt.

Näheres zum Stand der Technik ist dem US-Patent Nr. 5,815,741 sowie EP 0 679 984 zu entnehmen.

US 5,815,741 betrifft ein Sucher für eine Videokamera mit einer Blickrichtung-Eingabe-Vorrichtung oder ein am Kopf montierbares Display mit einer Blickrichtung-Eingabe-Vorrichtung, wobei die Blickrichtung eines Beobachters des Displays detektiert wird, und wobei die Beobachtungsumstände für das Bild unter Verwendung der Blickrichtungsinformationen angepaßt werden.

EP 0 679 984 lehrt ein Anzeigegerät mit einer Anzeigeeinrichtung zur Anzeige von Bildinformationen für einen Betrachter, einer bei dem Betrachter anbringbaren Erfassungseinrichtung zur Erfassung einer Bewegung zumindest eines Teiles des Körpers. Die Erfassungseinrichtung kann eine Bildabtasteinrichtung zur Bereitstellung eines Bildes zumindest eines Teils des Körpers des Betrachters und eine Einrichtung zur Erfassung einer Bewegung des zumindest einen Teils des Körpers des Betrachters aus einem Bildsignal von der Bildabtasteinrichtung. Das Gerät kann eine Anzeigeeinrichtung sowie eine Einrichtung zur Bereitstellung einer Ansicht der Umgebung des Benutzers über die Anzeige hinaus umfassen.

### Zusammenfassung der Erfindung

Die Erfindung sieht ein System zum Zurverfügungstellen von Informationen gemäß Anspruch 1 vor. Bevorzugte Ausführungsformen der Erfindung sind in den Unteransprüchen beschrieben.

Die beanspruchte Erfindung läßt sich durch die In dieser Schrift, d.h. in der vorliegenden Beschreibung sowie in den Zeichnungen, beschriebenen und dargestellten Ausführungsbeispiele besser verstehen. Im allgemeinen spiegelt die vorliegende Offenbarung bevorzugte Ausführungsbeisplele der Erfindung. Dem aufmerksamen Leser wird jedoch auffallen, daß einige Aspekte der beschriebenen Ausführungsbelsplele über den Schutzumfang der Ansprüche hinausragen. Sofern die beschriebenen Ausführungsbeisplele tatsächlich über den Schutzumfang der Ansprüche hinausragen, sind die beschriebenen Ausführungsbeispiele als zusätzliches Hintergrundinformation zu betrachten und stellen keine Definition der Erfindung per se dar.

Das Ziel der vorliegenden Offenbarung liegt darin, ein Informationssystem zur Verfügung zu stellen, dessen Informationsdarbeitung den natürlichen Bedürfnissen eines sehenden Menschen auf bisher unerreichte Art und Weise nachkommt. Es ist ein weiteres Ziel der vorliegenden Offenbarung , ein derartiges Informationssystem zu schaffen, das gengenüber dem Stand der Technik hinsichtlich Implementierbarkeit und Wirtschaftlichkeit verbessert worden ist. Ziel der vorliegenden Offenbarung ist auch, entsprechende Verfahren zum Zurverfügungstellen von Informationen zu schaffen.

In seiner allgemeinsten Form umfaßt das Informationssystem gemäß der vorliegenden Offenbarung eine Signalerfassungsvorrichtung, die von einem eine Netzhaut aufweisenden Auge zurückreflektierte Signale erfaßt, eine Informationsvorrichtung und eine Ausgabevorrichtung, die in Zusammenarbeit mit der Informationsvorrichtung Informationen in Korrelation mit den erfaßten Signalen zur Verfügung stellt. Bevorzugt werden die Informationen in Abhängigkeit von den erfaßten Signalen und/oder in Abhängigkeit von aus dem natürlich wahrgenommenen Gesichtsfeld. erfaßtem, sichtbarem Licht zur Verfügung gestellt.

Als Signalerfassungsvorrichtung dient vorzugsweise eines der oben besprochenen Brillensysteme, bei dem eine scannende Abtastvorrichtung ein Netzhautreflexbild der Netzhaut mindestens teilweise erfaßt. Eine Abwandlung dieser Abtastvorrichtung, die an der Hornhaut des Auges reflektiertes Licht anstelle des Netzhautreflexbildes erfaßt, ist insbesondere bei Infrarotanwendungen vorteilhaft, da die Hornhaut Licht mit einer Wellenlänge von ca. 1,1 µm stark reflektiert. Auch über die Erfassung der chemischen Veränderung der Stäbchen und/oder Zäpfchen ist es grundsätzlich möglich, entsprechend verwertbare Aussagen über das auf die Netzhaut einfallende Bild zu machen.

Die Erfinder dieser Erfindung haben auch festgestellt, daß eine der Erfassung aus dem Auge zurückreflektierter Signale komplementäre Erfassung des Gesichtsfeldes besondere Vorteile mit sich bringt. Zwecks einer solchen komplementären Erfassung umfaßt die Gesichtsfelderfassungsvorrichtung und/oder die Informationsvorrichtung des Informationssystems gemäß der vorliegenden Offenbarung bevorzugt eine im wesentlichen konfokal zum Auge angeordnete, spärisch oder spärisch wirkende Reflektionsschicht, die ein Teil des auf das Auge gerichteten Lichtes in eine Sensorvorrichtung zur Erfassung ablenkt. Aufgrund des im Vergleich zum Netzhaut- oder Hornhautreflex um ein Vielfaches höheren Reflektionsgrads der Reflektionsschicht wird bei gleich empfindlichen Photosensoren ein wesentlicher Lichtgewinn erzielt. Auch ließen sich entsprechend kostengünstige Photosensoren in der Sensorvorrichtung verwenden. Es kann also Vorteile mit sich bringen, wenn das auf das Auge fallende Licht nicht nur, nur teilweise oder gar nicht über das Netzhautreflex erfaßt wird.

Je nach Zielanwendung müssen nicht sämtliche räumliche Bereiche des Gesichtsfelds erfaßt werden. Beispielsweise bei einer Anwendung, bei der Zusatzinformationen bezüglich eines mit dem Auge angepeilten Objektes durch das Informationssystem zur Verfügung gestellt werden, könnte es ausweichen, das auf die Netzhautgrube (Fovea) fallende Licht zu erfassen und einer Mustererkennung oder sonstiger Analyse zu unterziehen. Denn ein mit dem Auge angepeiltes Objekt wird typischerweise auf die Netzhautgrube, die den Bereich des schärfsten Sehens darstellt, abgebildet. Somit wäre die Erfassung des auf diesen Teil der Netzhaut fallenden Lichtes möglicherweise ausreichend, um genügend viele charakterisierende Objektmerkmale ermitteln zu können.

Sinnvoll ist auch, wenn nur ein beschränkter Spektralbereich des auf das Auge fallenden Lichtes erfaßt wird. Wird, zum Beispiel das auf ein Auge fallende Infrarotlicht erfaßt, so kann auch bei Nacht die Orientierung des Auge bestimmt und/oder wertvolle Informationen aus dem Gesichtsfeld gewonnen werden.

Dementsprechend können jegliche Einschränkungen bezüglich der Erfassung des auf ein Auge fallenden Lichtes sinnvoll sein. Insbesondere werden Einschränkungen des erfaßten Spektralbereiches, des erfaßten Gesichtsfeldbereiches und der erfaßten Sehzeitabschnitte ggf. angewandt.

Zwecks der redundanten oder stereoskopischen Bilderfassung kann die dafür bestimmte Vorrichtung des Informationssystems derart ausgelegt sein, das auf mehrere Augen fallende Licht zu erfassen. Je nach Anwendungsgebiet müssen die Augen nicht zwangläufig einem einzelnen Person gehören. Zum Beispiel wäre es möglich, die von den Augen mehrerer Feuerwehrmänner wahrgenommenen Bilder zuzüglich Positions- und aus einer Infrarotspektralanalyse der Bilder ermittelten Brandstärkeninformationen auf Monitore in einer Einsatzzentrale einzuspielen.

In der Ophthamologie wird zwischen den Begriffen "Gesichtsfeld" und "Blickfeld" unterschieden. Ein Gesichtsfeld ist der Teil eines Raumes, der mit unbewegtem Auge erfaßt werden kann. Ein Blickfeld ist das Gebiet, das mit den Augen erfaßt werden kann. Somit ist hier, wie im übrigen, das Gesichtsfeld als Verursacher des auf ein Auge natürlich fallenden Lichtes zu verstehen.

### Die Gesichtsfelderfassung

Aufgrund der Einbindung einer Gesichtsfelderfassungsvorrichtung ist das Informationssystem in der Lage, sichtbares Licht aus dem dem Auge zugeordneten Gesichtsfeld in eine Qualität, d.h. mit einer Empfindlichkeit, einer Auflösung, einer Schärfe, u.s.w., zu erfassen, die die natürliche Wahrnehmung des Auges bei weitem übersteigt. Zudem ist es durch die in dieser Schrift beschriebene Korrelation des Informationzurverfügungstellens mit den von der Signalerfassungsvorrichtung erfaßten Signalen möglich, entsprechende Teile des erfaßten Lichts bei einer im Laufe des Infomationzurverfügungstellens auftretende Bearbeitung so zu behandeln, als wären sie aus dem Auge erfaßte Reflexbilder, d.h. als wären sie das tatsächlich Gesehene. Eine derartige Ausführung des Informationssystems kombiniert somit die Vorteile eines Informationssystem, das hochwertige Gesichtsfeldinformationen direkt aus dem Gesichtsfeld gewinnt, mit den Vorteilen eines Informationssystems, das tatsächlich gesehene Gesichtsfeldinformationen aus dem Auge gewinnt.

Die Korrelation des Informationzurverfügungstellens mit den aus dem Auge zurückreflektierten, erfaßten Signalen kann beispielsweise dadurch erfolgen, daß mehrere Bildpunkte eines Augenreflexbildes, z.B. eines Hornhaut- oder Netzhautreflexbildes, erfaßt werden, die über eine Auswertevorrichtung mit entsprechenden Bildpunkten aus dem erfaßten Gesichtsfeld in Verbindung gebracht werden. Auch eine über die erfaßten Signale festgestellte Blickrichtung des Auges kann dazu dienen, eine Korrelation zwischen aus dem erfaßten Gesichtsfeld gewonnenen Gesichtsfeldinformationen und dem tatsächlich Gesehenen zu schaffen. Wie unten beschrieben wird, kann die Korrelation jedoch auch darin bestehen, gewonnene Gesichtsfeldinformationen in eine mit dem Gesehenen korrelierte Art und Weise auf die Retina zu projizieren.

Selbstverständlich muß die Gesichtfelderfassungsvorrichtung nicht auf eine Erfassung des Gesichtsfeldes beschränkt sein, sondern kann auch eine teilweise oder komplette Erfassung des Blickfeldes umfassen, die eine mindestens partielle Erfassung des Gesichtsfeldes beinhaltet.

Die hohe Qualität des aus dem Blick- bzw. Gesichtsfeld erfaßten Bildes kann auch als Grundlage für eine übersensorische Informationsdarbietung dienen. Zum Beispiel könnten Gesichtsfeldinformationen aus dem erfaßten Blickfeldlicht derart gewonnenen und auf die Netzhaut projiziert werden, daß das vom Auge wahrgenommene Bild mindestens teilweise schärfer, näher, weitwinkliger oder auf sonstige Art und Weise übersinnlich wirken.

Diese Schrift sieht eine Informationsquelle vor, die eine Datenbank, eine Sensorik, eine Informationsnetzanbindung und/oder eine Auswertevorrichtung umfassen kann.

Eine besonders interessante Ausführungsform umfaßt eine Sensorik als Informationsquelle. Denn hiermit kann eine übersinnliche Wahrnehmung in Verbindung mit dem Gesehenen gebracht werden. Bei dem erwähnten Beispiel des Suchens einer Elektroleitung könnte die Informationsquelle Magnetfeldsensoren umfassen, die in der Lage sind, metallische Leitung bezüglich eines bekannten Koordinatensystems, beispielsweise das erfaßte Blickfeld, zu lokalisieren. Somit wäre es zum Beispiel mittels geeigneter Bildverarbeitungsssoftware möglich, den Verlauf vorhanderer Elektroleitungen mittels einer wie in den oben erwähnten Patentanmeldungen beschriebenen Projektion eines Zusatzbildes auf das vom Auge gesehene Bild zu überlagern.

Sämtliche Arten von bekannten Sensoren eignen sich zur Anwendung als Informationsquelle, insbesondere dann, wenn der Sensor anhand des erfaßten Lichtbildes aktiviert bzw. abgefragt wird. Zum Beispiel wäre es bei der Prüfung einer integrierten elektronischen Schaltung möglich, daß nach gezielten Anblick einer Leitung auf einem Schaltplan der Schaltung und einem Tastensdruck die Position dieser Leitung auf einem fertigen Chip berechnet wird, so daß die Strom- und Spannungswerte der Leitung mittels einer berührungslosen Meßgerät erfaßt und dem Anwender über das Brillensystem dargestellt werden.

Ein Beispiel für ein eine Datenbank und eine Informationsnetzanbindung umfassendes Informationssystem wäre ein betriebsinternes Postverteilungssystem, bei dem Akten mit Barcodeaufklebern versehen sind, die die jeweilige Akte eindeutig kennzeichnen. Soll eine Akte betriebsintern verschickt werden, gibt der Absender beispielsweise die Durchwahl des Empfängers und einen die Akte bezeichnenen Code mittels einer Software ein, die diese Daten in einer Datenbank auf einer der vielen bekannten Weisen entsprechend abspeichert. Bei einer späteren Sortierung der Akte wird der kennzeichnende Barcode über das einem Postverteilungsangestellten getragene Brillensystem beispielsweise bei gezieltem Blick und Tastenklick erfaßt und durch eine Erkennungsvorrichtung oder -software erkannt. Per Funkverbindung mit einem betriebsinternen Datennetz werden die der Akte zugeordneten, postverteilungsrelevanten Daten aus der Datenbank geholt und über eine geeignete Ausgabevorrichtungen nach evtl. Aufbereitung an den Postverteilungsangestellten, beispielsweise als Ansage über Kopfhörer "Hr. Schmidt, Finanzwesen, Gebäude G, 3. Stock, Zimmer 310", mitgeteilt.

Unter Auswertevorrichtung sind sämtliche Arten von Auswertevorrichtungen zu verstehen, insbesondere Bildverarbeitungsvorrichtungen. Solche Auswertevorrichtungen sind auch in den obigen Beispielen zur Sprache gekommen.

Gemäß der vorliegenden Offenbarung können die Informationen taktil, visuell, hörbar, riechbar und/oder geschmacklich zur Verfügung gestellt werden. Es gehört zur Aufgabe der vorliegenden Offenbarung, eine Informationsdarbietung zu ermöglichen, die den Bedürfnissen eines sehenden Menschens auf bisher unerreichte Art und Weise nachzukommen. Hierzu kann gehören, daß die Informationen dem Menschen in geeigneter Weise, das heißt unter Ausnutzung eines oder mehrerer der fünf Sinne, zur Verfügung gestellt werden können. Die Informationen können jedoch auf beliebige Art und Weise zur Verfügung gestellt werden und bedürfen keinen bestimmten Adressant. Beispielsweise können die Informationen einem weiteren System zur Vefügung gestellt werden oder durch eine optische oder akustische Ausgabevorrichtung in die Umgebung ausgestrahlt werden. Schon durch die in dieser Schrift beschriebene Abhängigkeit zwischen dem Zurverfügungstellen von Informationen und dem auf das Auge fallenden Lichtbild wird erreicht, daß der vom sehenden Menschen erwartete Zusammenhang zwischen Gesehenem und zur Verfügung gestellten Informationen besteht.

Diese Abhängigkeit wird gemäß der vorliegenden Offenbarung von der Vorrichtung bei der Ermittlung der Informationen, bei dem Zurverfügungstellen der Information oder während beider dieser inhärenten Vorgängen berücksichtigt. Beispiele für eine Berücksichtigung dieser Abhängigkeit bei der Ermittlung der Informationen sind oben angegeben. Beim Zurverfügungstellen der Informationen kann diese Abhängigkeit zum Beispiel dadurch berücksichtigt werden, daß die Informationen mittels einer Rückprojektion in das Auge auf eine Art und Weise in das gesehene Bild eingeblendet werden, daß ein zeitlicher, farblicher, räumlicher, kontrastbezogener, oder sonstiger sinnvoller Zusammenhang zwischen den Informationen und dem gesehenen Bild hergestellt wird. Insbesondere kann die Abhängigkeit darin bestehen, daß das erfaßte Lichtbild dazu verwendet wird, die Lage und Orientierung des Augapfels festzustellen, so daß ein zwecks eines Zurverfügungstellens der Informationen auf das Auge projiziertes Bild bei einer Bewegung des Auges festzustehen scheint, sich bei einer Bewegung des Auges mitzubewegen scheint oder sich auch bei einer Bewegung des Auges entsprechend einem vorgegebenen Verlauf zu bewegen scheint. Insbesondere läßt sich die Auswirkung der Sakkadenbewegungen des Auges auf diese Vorgänge berücksichtigen bzw. kompensieren.

Selbstverständlich müssen die Informationen nicht unbedingt einem Menschen sondern können auch einem anderen System zur Verfügung gestellt werden.

### Tracking

Es ist also mit Informationssystem gemäß der vorliegender Offenbarung möglich, die Lage und Ausrichtung mindestens eines Auges schnell, genau und mit geringem Aufwand zu ermitteln, z.B. mit einer Bestimmungsrate von 100 Hz, einer Positionsgenauigkeit von wenigen Mikrometern und einer Vorrichtung in tragbarer Bauweise. Unter Anwendung des Informationssystems bei der dynamischen Bestimmung der Orientierung des Auges kann die Verarbeitung derart rasch erfolgen, daß die Genauigkeit durch die Sakkadenbewegungen des Auges nicht verfälscht wird. Dies wird dadurch erreicht, daß das Informationsystem eine das Auge nicht berührende Signalerfassungsvorrichtung aufweist, die vom Auge zurückreflektierte Signale erfaßt. Reflektierbare Signale, zum Beispiel Schall- oder elektromagnetische Signale, erlauben eine hochfrequente Erfassung, so daß die Verarbeitungsgeschwindigkeit hauptsächlich von einer vom Informationssystem umfaßten Auswertevorrichtung bestimmt wird. Auf dem Gebiet der signalverarbeitenden Hardware sind jedoch erhebliche Fortschritte in den letzten Jahren bezüglich der Arbeitsgeschwindigkeit, des Stromverbrauchs und der Systemgröße erzielt worden. Die Erforschungen der Erfinder haben ergeben, daß ein Informationssystem durch eine derartige Gestaltung die erstrebte Verarbeitungsgeschwindigkeit erreichen kann.

Typischerweise dient ein Teil des Informationssystems selbst als Referenzkoordinatensystem. Allerdings ist auch möglich, daß das Informationssystem lediglich ein Bezugskoordinatensystem in einem anderen Referenzkoordinatensystem darstellt, und daß das Verhältnis zwischen dem Bezugskoordinatensystem und dem Referenzkoordinatensystem beispielsweise durch die Auswertevorrichtung oder einen anderen Mechanismus ermittelt wird.

Bevorzugt erfaßt die Signalerfassungsvorrichtung vom Auge zurückreflektiertes Licht. Licht bildet ein vorzügliches Medium zur Übertragung der vom Auge zurückreflektierte Signale, da die Einsatzfähigkeit des Auge ein Vorhandensein von Licht voraussetzt. Allerdings ergibt sich eine Überlagerung der vom Licht aus dem Gesichtsfeld übertragenen Gesichtsfeldsignalinformationen mit der Augenreflexsignalinformation, die durch die Reflektion am Auge entsteht. Diese unterschiedlichen Informationen lassen sich jedoch unter Anwendung bekannter Signalverarbeitungsmethoden unterscheiden und sinnvoll zur Bestimmung der Orientierung des Auge verwenden. Dies ist insbesondere dann der Fall, wenn das Signalübertragungsmedium aus einer zum Informationssystem gehörenden Signalquelle stammt, das das Medium vor seiner Reflektion am Auge mit einem vorgegebenen Signal beaufschlagt.

Ähnlich kann die Erfassung von Signalen aus anderen Signalübertragungsmedien als Licht auch vorteilhaft sein. Zum Beispiel sind Bauteile zur Erzeugung und Erfassung von Schallwellen in verschiedenen kostengünstigen und kompakten Auführungen am Markt erhältlich. Solche Bauteile lassen sich auch als integrierte Elemente einer integrierten Schaltung verwirklichen. Ähnliche Überlegungen gelten den nicht sichtbaren Frequenzbereichen von elektromagnetischen Wellen.

Obwohl nicht vollständig erforscht, ist es denkbar, daß ein Informationssystem mit einer Mehrzahl an Signalerfassungsvorrichtungen, die Signale aus unterschiedlichen Medien oder Spektralbereichen erfassen, verbesserte Systemeigenschaften aufweisen könnte. Diese Erkenntnis liegt den Überlegungen zugrunde, daß die Auswertevorrichtung im Falle einer Unterbelastung auch andere Systemaufgaben übernehmen könnte, und daß die von der Auswertevorrichtung vorgenommene Signalbearbeitung stark vom Informationsgehalt des zu bearbeitenden Signals abhängt. So brächte es Vorteile, das Informationssystem auf einer Signalerfassung zu basieren, die zur Bewertung nur wenig Arbeitsleitung von der Auswertevorrichtung beansprucht, jedoch allein evtl. nicht die Basis für eine ausreichende Genauigkeit liefert, und diese bearbeitungsarme Signalerfassung derart durch die Ergebnisse einer genauen und bearbeitungsintensiven, jedoch nur intermittierend durchzuführenden Signalerfassung zu ergänzen bzw. kalibrieren, daß die notwendige Genauigkeit zu jeder Zeit erreicht wird.

Sinnvoll hat sich eine Netzhautreflexerfassung erwiesen, bei dem das Netzhautreflex natürlichen oder künstlichen Lichts als aus dem Auge zurückreflektiertes Signal intermittierend oder partiell erfaßt wird. Eine vollständige Erfassung des Netzhautreflexes ist sowohl zeit- als auch arbeitsintensiv. Andererseits ist eine Erfassung des Netzhautreflexes insofern sinnvoll, als sie eine direkte Ermittlung des wahrgenommen Gesichtsfelds in Relation zur Netzhaut erlaubt. Denn, wie oben erwähnt, eine Aufbereitung des erfaßten Netzhautreflexes läßt sowohl Netzhautmerkmale, wie z.B. die Fovea centralis oder der blinde Fleck, als auch das Reflexbild des auf das Auge fallenden Lichts erkennen. Auch das in der Aderhaut vorhandene Blutgefäßnetz wird bei entsprechender Aufbereitung des Netzhautreflexbildes sichtbar, was eine sehr gute Grundlage zur Bestimmung der Orientierung des Augapfels liefert. Wird das Netzhautreflex deshalb intermittierend oder partiell erfaßt, so läßt sich der Bearbeitungsaufwand reduzieren, während auf eine genaue Ermittlung der Relation des wahrgenommenen Gesichtsfelds zur Netzhaut nicht verzichtet wird. Selbstverständlich lassen sich Netzhautmerkmale ohne eine Netzhautreflexerfassung verfolgen. Beispielsweise lassen sich die Blutgefäße der Aderhaut über ihren im Infrarotbereich sichtbaren Wärmeausstrahlung erkennen.

Die vorliegende Erfindung wird nachstehend anhand der Beschreibung von Ausführungsbeispielen unter Bezugnahme auf die Zeichnung näher beschrieben. Es werden viele Merkmale der vorliegenden Offenbarung im engen Zusammenhang des jeweiligen, konkret dargestellten Ausführungsbeispiels erläutert. Selbstverständlich läßt sich jedes einzelnen Merkmal der vorliegenden Offenbarung mit jedem anderen Merkmal kombinieren, soweit die resultierende Kombination nicht zu einem für den Fachmann als sofort unsinnig erkennbaren Ergebnis führt.

Es zeigen:
Figur 1 ein Informationssystem gemäß einem ersten Ausführungsbeispiel der vorliegenden Offenbarung ;
Figur 2 eine detaillierte Ansicht eines Auges im Querschnitt;
Figur 3 eine bekannte Ausführungsform eines interaktiven Brillensystems, bei der eine Signalerfassungsvorrichtung in Form einer scannenden Augenabtastvorrichtung vorgesehen ist;
Figur 4 eine bekannte Ausführungsform einer interaktiven Brille, bei der eine Ausgabevorrichtung in Form einer scannenden Projektionsvorrichtung vorgesehen ist;
Figur 5A eine interaktive Brille gemäß einem ersten Ausführungsbeispiel;
Figur 5B eine Detailzeichung einer in der Figur 5 dargestellten kombinierten Signalerfassungs- und Projektionsvorrichtung;
Figur 6A eine interaktive Brille gemäß einem zweiten Ausführungsbeispiel;
Figur 6B eine Detailzeichung einer in der Figur 6A dargestellten kombinierten Signalerfassungs- und Projektionsvorrichtung;
Figur 7A eine interaktive Brille gemäß einem dritten Ausführungsbeispiel;
Figur 7B eine Detailzeichung einer in der Figur 7A dargestellten kombinierten Signalerfassungs- und Projektionsvorrichtung;
Figur 8 eine interaktive Brille gemäß einem vierten Ausführungsbeispiel;
Figur 9 eine interaktive Brille gemäß einem fünften Ausführungsbeispiel;
Figur 10A eine Draufsicht einer Brille gemäß einem sechsten Ausführungsbeispiel;
Figur 10B eine Frontansicht einer Brille gemäß einem sechsten Ausführungsbeispiel;
Figur 11A das natürlich wahrgenommene Gesichtsfeld eines Benutzers eines gemäß einem siebten Ausführungsbeispiel gestalteten Informationssystems;
Figur 11B das natürlich wahrgenommene Gesichtsfeld eines Benutzers eines gemäß einem siebten Ausführungsbeispiel gestalteten Informationssystems;
Figur 11C eine schemenhafte Darstellung eines Abtastmusters;
Figur 11D eine schemenhafte Darstellung eines abgeänderten Abtastmuster;
Figur 12A das natürlich wahrgenommene Gesichtsfeld eines Benutzers eines gemäß einem achten Ausführungsbeispiel gestalteten Informationssystems;
Figur 12B das natürlich wahrgenommene Gesichtsfeld eines Benutzers eines gemäß einem achten Ausführungsbeispiel gestalteten Informationssystems;
Figur 12C das natürlich wahrgenommene Gesichtsfeld eines Benutzers eines gemäß einem achten Ausführungsbeispiel gestalteten Informationssystems;
Figur 12D das natürlich wahrgenommene Gesichtsfeld eines Benutzers eines gemäß einem achten Ausführungsbeispiel gestalteten Informationssystems;
Figur 12E das natürlich wahrgenommene Gesichtsfeld eines Benutzers eines gemäß einem achten Ausführungsbeispiel gestalteten Informationssystems;
Figur 13A das natürlich wahrgenommene Gesichtsfeld eines Benutzers eines gemäß einem neunten Ausführungsbeispiel gestalteten Informationssystems;
Figur 13B das natürlich wahrgenommen Gesichtsfeld eines Benutzers eines gemäß einem neunten Ausführungsbeispiel gestalteten Informationssystems;
Figur 14A ein Informationssystem gemäß einem zehnten Ausführungsbeispiel;
Figur 14B ein Informationssystem gemäß einem zehnten Ausführungsbeispiel;
Die Figur 15 ein Informationssystem gemäß einem elften Ausführungsbeispiel;
Die Figur 16 eine schematische Darstellung eines Informationssystems gemäß einem zwölften Ausführungsbeispiel; und
Figure 17 ein optisches System gemäß einem dreizehnten Ausführungsbeispiel.

In der Beschreibung der Figuren werden ähnliche oder identische Gegenstände mit ähnlich oder gleich endenden Bezugsziffern bezeichnet. Viele der abgebildeten Gegenstände weisen symmetrische oder komplementäre Komponenten auf, die durch einen Zusatzbuchstaben, beispielsweise "L" für links und "R" für rechts, nach dem Bezugsziffer unterschieden werden. Betrifft die Aussage jede einzelne Komponente einer solchen symmetrische oder komplementären Gruppierung, wird lauf den Zusatzbuchstaben in manchen Fällen der Übersichtlichkeit halber verzichtet.

### Figur 1

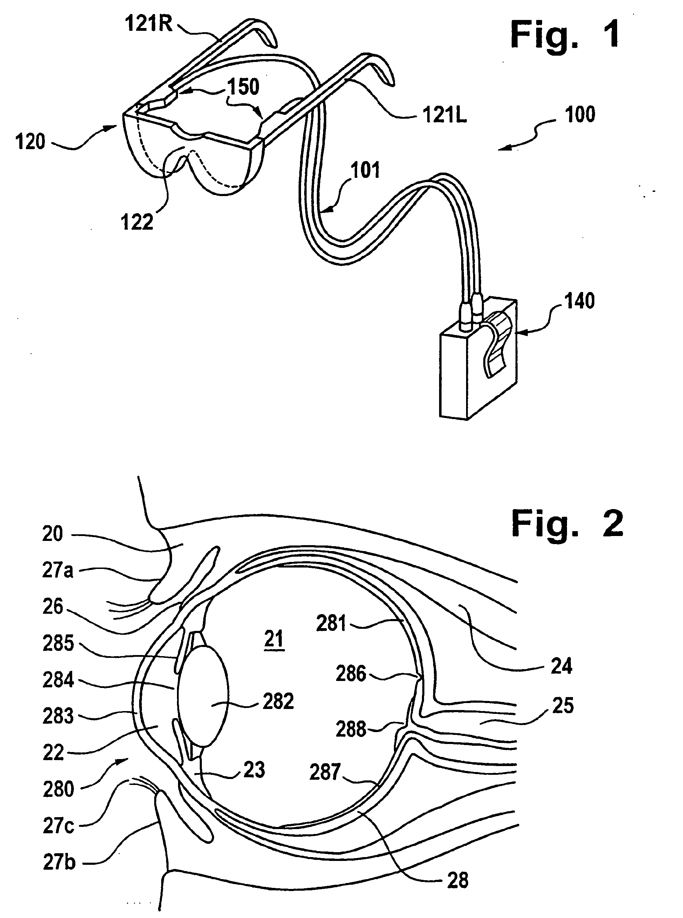

Figur 1 zeigt ein Informationssystem 100 gemäß einem ersten Ausführungsbeispiel der vorliegenden Offenbarung. Das Informationsystem 100 ist in Form eines interaktiven Brillensystems 120 bzw. einer interaktiven Brille 120 ausgeführt, die zwei optische Vorrichtungen 150 umfaßt. Bevorzugt befinden sich die optischen Vorrichtungen 150 jeweilig auf einer Innenseite eines linken 121L oder rechten 121R Bügelteils der Brille 120. Je nach Anwendungsbereich sind auch andere, die Sicht nicht störende Anordnungen der optischen Vorrichtungen, z.B. im Bereich eines über die Nasenwurzel eines Beneutzers verlaufenden Nasenstegs 122 der Brille 120, sinnvoll.

Die optische Vorrichtung 150 der Brille 120 ist über Verbindungsleitungen 101 an eine Prozessoreinheit 140 angeschlossen. Sind Photodetektoren und/oder Lichtquellen von den optischen Vorrichtungen umfaßt, dienen die Verbindungsleitungen zur Übertragung von elektrischen Detektor- bzw. Steuersignale. Die Photodetektoren bzw. Lichtquellen können jedoch in der Prozessoreinheit 140 angeordnet und über lichtleitende Verbindungsleitungen 101 an die optischen Vorrichtungen 150 der Brille 120 angeschlossen werden. Dies trägt zur Gewichtsreduktion der Brille 120 bei.

### Figur 2

Figur 2 zeigt zwecks Verständnis der vorliegenden Offenbarung eine detaillierte Ansicht eines Auges 280 im Querschnitt. Das Auge 280, das in einer aus Schädelknochen gebildeten Augenhöhle 20 (lat. Orbita) im Kopf eines Menschen untergebracht und hier im Sinne eines Augapfels 280 zu verstehen ist, besteht aus einer von einer lichtdurchlässigen Hornhaut 283 (lat. Kornea) und einer sichtlich weißen Lederhaut 28 (lat. Sklera) umgebenen Kammer. Die Lederhaut 28 ist auf seiner dem Inneren des Auges 280 zugewandten Seite von einer Aderhaut 287 (lat. Choroidea) überzogen, die auf seiner ebenfalls inneren Seite eine lichtempfindliche Netzhaut 281 (lat. Retina) trägt und diese mit Blut versorgt. Durch ihre Pigmentierung verhindert die Aderhaut 287 eine Steuung des darauffallenden Lichts, die das Sehvermögen stören könnte.

Das Gewebe der Netzhaut 281 umfaßt zwei Arten von Photorezeptorzellen, nämlich Stäbchen und Zapfen (beide nicht dargestellt), die dem Menschen den Sehsinn ermöglichen. Diese Photorezeptorzellen absorbieren das durch eine Augenlinse 282 gebündelte Licht in einem Wellenlängenbereich von ca. 380-760 nm und verwandeln es durch eine Reihe von chemischen Reaktionen in elektrische Nervensignale. Die Signale der verschiedenen Nervenzellen der Netzhaut 281 werden dann über einen Sehnerv 25 an das Gehirn weitergeleitet und dort zu einem wahrnehmbaren Bild verarbeitet. Die zahlreichen, ca. 120 Millionen zählenden und stark lichtempfindlichen Stäbchen sind auf die Signalaufnahme im Dämmerlicht (sogenanntes skotopisches Sehen) spezialisiert und liefern ein Graustufenbild. Die ca. 6,5 Millionen, vergleichsweise weniger lichtempfindlichen Zapfen dagegen sind für das Farbsehen bei Tageslicht (sogenanntes photopisches Sehen) zuständig. Bei der Lichtabsorbtion findet eine Oxidierung von Pigmenten in den Photorezeptorenzellen statt. Zur Regenerierung der Pigmente bedarf es bei den Zapfen ca. 6 Minuten und bei den Stäbchen ca. 30 Minuten. Eine Betrachtungsdauer von ca. 200 msec ist notwendig, bis der Sehreiz über die Photorezeptoren einsetzt und eine Informationsaufnahme über die Netzhaut 281 erfolgt.

Die Netzhaut 281 weist eine Vertiefung 286 auf, die durch ihre im Vergleich zur übrigen Netzhaut höher Dichte an Zapfen als etwas stärker pigmentiert erscheint. Diese Vertiefung 286, die üblicherweise Sehgrube 286 (Fovea centralis) genannt wird, liegt in einem als "gelber Fleck" (lat. Makula) bekannten Bereich der Netzhaut und stellt den Bereich des schärfsten Sehens dar. Die Fovea centralis 286 ist nur mit Zapfen besetzt, weist eine sehr hohe Zapfendichte auf und beansprucht lediglich ca. 0,01% der Netzhautoberfläche. An der mit dem Bezugszeichen 288 gekennzeichneten Stelle vis-à-vis der Linse 282 tritt das Sehnerv 25 durch eine siebartige Öffnung in der Lederhaut 28 in das Innere des Auges ein. Diese Stelle 288 weist keine Photorezeptorzellen auf, weshalb sie als "blinder Fleck" bezeichnet wird.

Die von der Hornhaut 283 und der Lederhaut 28 gebildeten Kammer ist durch eine verformbare Linse 282 und einen muskelösen Strahlenkörper 23 (auch Ziliarkörper genannt), der die Linse 282 trägt; unterteilt. Der zwischen der Linse 282 und der Netzhaut 281 liegende Teil 1 der Kammer, der ca. 2/3 des Augapfels ausmacht, bildet einen sogenannten Glaskörper 21, ein gallertiges Gebilde, das zu über 98% aus Wasser besteht und die Netzhaut 281 stützt und schützt. Der als Vorderkammer 22 bezeichnete, zwischen der Hornhaut 283 und der Linse 282 liegende Teil der Kammer enthält eine Flüssigkeit, die die Hornhaut 283 ernährt. In ihrer Urform bricht die Linse 282 das auf das Auge fallende Licht typischerweise derart, daß das ferne Gesichtsfeld auf die Netzhaut 281 scharf abgebildet wird. Durch Anspannung/Entspannung der Muskeln des Ziliarkörper 23 kann die Form und somit auch die Brechungscharakteristik der Linse 282 über einen breiten Bereich verändert werden, um beispielsweise eine scharfe Abbildung nahliegender Gegenstände des Gesichtsfelds auf die Netzhaut 281 zu ermöglichen. Dieser Vorgang läuft in den meisten Fällen für den betroffenen Menschen unbewußt ab.

Unmittelbar vor der Linse 282 befindet sich in der Vorderkammer 22 eine aus gefärbtem Gewebe bestehende Blende 285 veränderbaren Durchmessers, die den Lichteinfall auf die lichtempfindlichen Teile des Auges 280 reguliert und dem Auge 280 seine charakteristische Färbung verleiht. Diese Blende 285 wird deshalb als Regenbogenhaut 285 (lat. Iris) bezeichnet. Aufgrund der geringen Lichtrückstrahlung der Linse 282, des Glaskörpers 21 und der Netzhaut 281 erscheint der zentrale Bereich der Iris 285 schwarz und wird Pupille 284 bezeichnet. Auch die Regulierung der Pupillengröße läuft für den Menschen unbewußt ab.

Das Auge 280 ist über sechs teils parallel, teils schräg zueinander verlaufende Muskeln 24 an die Schädel verbunden, die ein Schwenken des Auges 280 und folglich eine Änderung der Blickrichtung ermöglichen. Das binokular, ohne Bewegung der Augen 280 erfaßte Gesichtsfeld umfaßt horizontal ca. 170° und vertikal ca. 110°. Werden die Augen 280 bewegt, kann ein binokulares Blickfeld von horizontal ca. 290° und vertikal ca. 190° erfaßt werden. Der von der Fovea centralis 286 erfaßten Bereich des schärften Sehens umfaßt lediglich ca. 1°. Eine fiktive Achse durch die Mitte dieses Bereichs wird als Sehachse bezeichnet und entspricht der Blickrichtung. Auch eine Rotation des Auges um die Sehachse wird durch die Muskeln 24 ermöglicht.

Die sechs Muskeln 24 sind für sämtliche Augenbewegungen zuständig. Bei einer Betrachtung eines Fixpunkts finden sogenannte Mikrotremors des Auges 280 statt, bei denen das Augen 280 leicht zittert, um eine vorübergehende Erschöpfung der chemischen Reaktionfähigkeit der betroffenen Photorezeptorzellen beim gleichbleibenden Reiz zu vermeiden. Während eines Blickrichtungswechsels oder einer Kopfbewegung finden sogenannte Sakkadenbewegungen statt, mit deren Hilfe die Fovea centralis 286 auf ihr neues Fixationsziel gerichtet bzw. auf ihr bisheriges Fixationsziel gehalten wird. Bei dieser sehr komplex ablaufenden Bewegung wird das Auge 280 unwillentlich mit einer kleinen Amplitude von bis zu mehreren zehn Grad und einer extrem schnellen Winkelgeschwindigkeit von bis zu mehreren hundert Grad pro Sekunde hin und her bewegt. Bei der Verfolgung eines sich bewegenden Objekts erreicht das Auge 280 Winkelgeschwindigkeiten von lediglich eins bis zwei hundert Grad pro Sekunden.

Zum Schutz des Augapfels 280 hat der Mensch bewegliche Hautfalten, nämlich ein Oberlid 27a und ein Unterlid 27b, die ein Schließen der Augenhöhle 20 gegen äußere Einflüsse ermöglicht. Die Lider 27a und 27b schließen sich reflektorisch bei einfallenden Fremdkörpern und starker Blendung. Darüber hinaus sorgen die Lider 27a und 27b durch regelmäßige, meist unwillkürlichen Lidschlag für einen gleichmäßig auf der Hornhaut 283 verteilten Tränenfilm, der die äußere Oberfläche der Hornhaut 283 vor einem Austrocknen wahrt und wäscht. Die Lider 27a und 27b weisen auch Wimpern 27c auf, die das Auge 280 ebenfalls vor Staub schützen. Eine Bindehaut 26 kleidet den Raum zwischen den Lidern 27a bzw. 27b, der Aughöhle 20 und dem Augapfel 280 aus. Die Bindehaut 26 geht einerseits in die Lidinnenseite über, andererseits in die Hornhaut 283, und stellt einen zweiten Schutzwall gegen das Eindringen von Keimen und Fremdkörpern dar.

### Figur 3

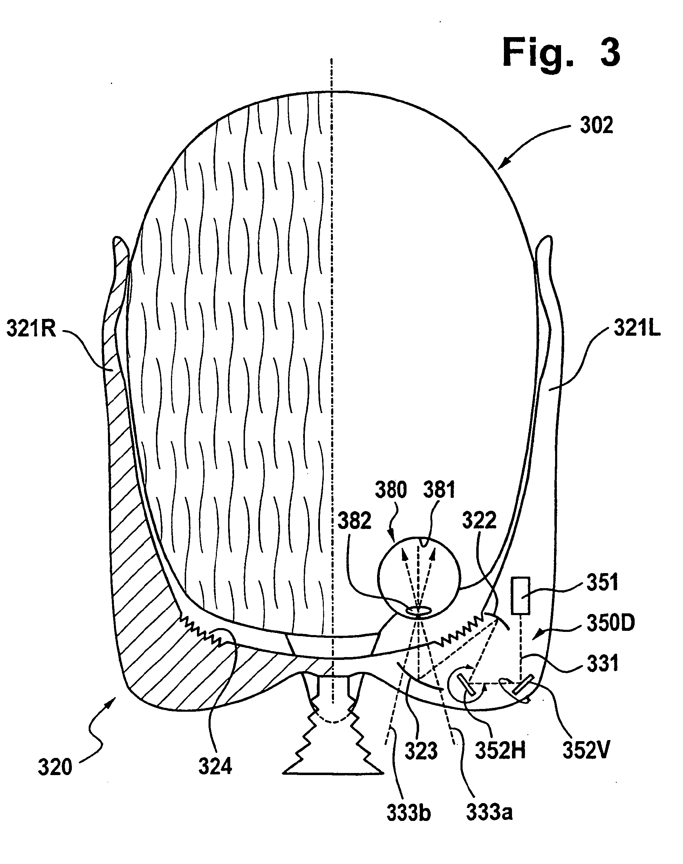

Figur 3 zeigt eine bekannte Ausführungsform des wie oben beschriebenen, interaktiven Brillensystems bzw. Brille 320, bei der eine Signalerfassungsvorrichtung in Form einer scannenden Augenabtastvorrichtung 350D vorgesehen ist. Dabei stellt die linke Bildhälfte eine Draufsicht auf den Kopf eines Benutzers 302 samt Brille 320 mit rechtem Bügelteil 321R dar, während die rechte Bildhälfte ein durch den linken Bügelteil 321L verlaufenden Querschnitt der Brille 320 wiedergibt. Außer der zur interaktiven Brille 320 gehörenden Vorrichtungen sind in der Figur 3 keine weiteren Komponenten der Informationssystem 100 abgebildet.

Gemäß der abgebildeten Ausführungsform werden auf das Auge 380 fallende Lichtstrahlen 333a und 333b, die beispielsweise aus dem Gesichtsfeld stammen, von der Linse 382 auf der Netzhaut 381 als zusammenhängendes Bild scharf abgebildet und von ihr als Netzhautreflexbild zurückreflektiert. Ein so zurückreflektierter Lichtstrahl 3311 passiert in ungekehrte Richtung erneut die Linse 382, wird über zwei, zum Spiegelsystem der Brille 320 gehörende konkave Spiegel 322 und 323 fokussiert und wie abgebildet auf eine scannende Augenabtastvorrichtung 350D gelenkt. Die Augenabtastvorrichtung 3500 umfaßt eine Signalerfassungsvorrichtung 351 in Form eines Fotodetektors 351, der den von der Netzhaut 381 zurückreflektierten Lichtstrahl 331 erfaßt, sowie zwei bewegliche Flachspiegel 352H und 352V, die eine horizontale bzw. vertikale Ablenkung des Lichtstrahls 331 auf den Fotodetektor 351 bewirken. Gemäß der Ausführung der Figur 3 umfaßt die Brille 320 zusätzlich eine Lichtfalle 324, die einen Lichteinfall aus unerwünschten Einfallsrichtungen verhindert. Zur Vereinfachung des Spiegelsystem der Brille 320 kann der Spiegel 323 durch eine verspiegelte Innenoberflache des Brillenglases verwirklicht werden. Allerdings muß die Oberfläche eine bestimmte Form aufweisen, um eine Erfassung des gesamten Netzhautreflexbildes auch bei einer eventuellen verdrehten Stellung des Auges 380 zu ermöglichen. Dies schränkt wiederum die Gestaltungsmöglichkeiten der Brille 320 ein.

Durch die Kombination eines punktförmigen Detektors 351 mit entsprechender Steuerung der Flachspiegel 352H und 352V erfolgt eine serielle punktuelle Abtastung des Netzhautreflexbildes als Bildpunktfolge. Bevorzugt wird die Netzhaut 381, wie in der DE 196 31 414 A1 und der DE 197 28 890 beschrieben, mit einem kreis-, spiral- oder ellipsenförmigen Scanmuster abgetastet. Dies hat den Vorteil, daß die Flachspiegel 352 ohne rückartigen Bewegungen angetrieben werden können, und daß eine höhere Bildpunktdichte (Anzahl' der Bildpunkte pro Flächeneinhelt der Netzhaut) 1m Bereich der Fovea centralis 286 sich erfassen läßt.

Dem Aufnahmevorgang vorgeschaltet wird soweit noch nicht in einem vorhergenden Projektionsvorgang geschehen - vorzugsweise ein geeigneter Synchronisationsvorgang zur Bestimmung der momentanen Sehachse, damit der Scanvorgang augenzentriert durchgeführt werden kann.

### Figur 4

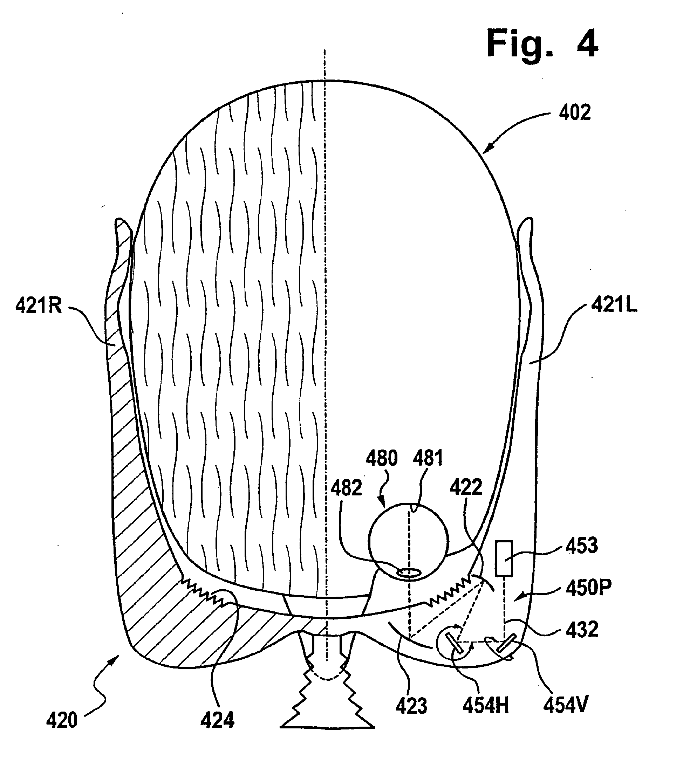

Figur 4 zeigt eine bekannte Ausführungsform der wie oben beschriebenen, interaktiven Brille 420, bei der eine Ausgabevorrichtung in Form einer scannenden Projektionsvorrichtung 450P vorgesehen ist. Dabei stellt die linke Bildhälfte eine Draufsicht auf den Kopf eines Benutzers 402 samt Brille 420 mit rechtem Bügelteil 421R dar, während die rechte Bildhälfte ein durch den linken Bügelteil 421L verlaufenden Querschnitt der Brille 420 wiedergibt. Außer der zur interaktiven Brille 420 gehörenden Vorrichtungen sind in der Figur 4 keine weiteren Komponenten der Informationssystem 100 abgebildet.

Gemäß der abgebildeten Ausführungsform umfaßt die scannende Projektionsvorrichtung 450P eine einen Projektionslichtstrahl 432 emittierende Lichtquelle 453, beispielsweise eine Laserdiode oder eine über ein Linsensystem fokusierte LED, sowie zwei bewegliche Flachspiegel 454H und 454V. Der Projektionslichtstrahl 432 wird über die beweglichen Flachspiegel 454H und 454V auf ein Spiegelsystem der Brille 420 gelenkt, das zwei konkave Spiegel 422 und 423 umfaßt, die den Projektionslichtstrahl 432 auf die Linse 482 eines Auges 480 und schließlich auf die Netzhaut 481 wirft. Zur Vereinfachung des Spiegelsystem der Brille 420 kann der Spiegel 423 durch eine verspiegelte Innenoberflache des Brillenglases verwirklicht werden. Allerdings muß die Oberfläche eine bestimmte Form aufweisen, um eine Projektion auf alle Bereiche der Netzhaut 481 auch bei einer eventuellen verdrehten Stellung des Auges 480 zu ermöglichen. Dies schränkt wiederum die Gestaltungsmöglichkeiten der Brille 420 ein. Zur Vermeidung störender Lichteinfälle läßt sich die Brille 420 mit einer Lichtfalle 424 ausstatten, die Lichteinfälle aus unerwünschten Einfallsrichtungen verhindert.

Durch die Kombination einer punktförmigen Lichtquelle 453 mit entsprechender Steuerung der Flachspiegel 452H und 452V, die jeweils eine horizontale bzw. vertikale Ablenkung des Projektionslichtstrahis 432 bewirken, erfolgt eine serielle punktuelle Projektion eines Bildes. Bevorzugt erfolgt die Projektion, wie in der DE 196 31 414 A1 und der DE 197 28 890 beschrieben, mit einem kreis-, spiral- oder ellipsenförmigen Scanmuster. Dies hat den Vorteil, daß die Flachspiegel 452 ohne rückartigen Bewegungen angetrieben werden können, und daß sich eine höhere Bildpunktdichte im Bereich der Fovea centralis 286 auf die Netzhaut 481 projizieren läßt.

### Projektion

Der Grad der Wahrnehmung eines in das Auge 480 projizierten Bildes kann im Verhältnis zum natürlich wahrgenommenen Bild durch die Helligkeit der projizierten Bildpunkte gesteuert werden. Allerdings ist die reti nal e Wahrnehmung ein zutiefst komplexer Vorgang, bei der auch psychologische Effekte eine sehr starke Rolle spielen. Hier wird auf die einschlägige fachliteratur verwiesen.

Vereinfacht läßt sich jedoch sagen, daß die Retina 481 sich auf die Helligkeit des insgesamt auf sie fallenden Lichtes einstellt. Es ist zum Beispiel bekannt, daß das geringe Leuchten der Uhr eines Radioweckers, das bei Tageslicht gar nicht wahrgenommen wird, bei dunkler Nacht ein ganzes Zimmer zu erleuchten scheinen kann. Andersherum ist das starke Scheinwerferlicht entgegenkommender Fahrzeuge bei Tageslicht kaum wahrnehmbar. Es wird also die Helligkeit eines einzelnen Bildpunktes in Relation zu den ansonsten wahrgenommenen Bildpunkte empfunden. Auch lokal betrachtet, funktioniert die Retina 481 ähnlich. Übersteigt die Helligkeit eines auf einen Gebiet der Retina 481 projizierten Bildpunkt die Helligkeit des ansonsten auf dieses Gebiet fallenden Lichtes um ca. 10%, so wird effektiv lediglich der projizierte Bildpunkt anstelle des sonstigen Lichts von diesem Gebiet der Retina 481 wahrgenommen. Aufgrund psychologischer Effekte kann der genaue Wert statt bei 10% auch zwischen 5%-10%, 10%-15% oder gar 15%-20% liegen.

Dem Projektionsvorgang vorgeschaltet wird - soweit noch nicht in einem vorhergenden Scanvorgang geschehen - bei Bedarf vorzugsweise ein geeigneter Synchronisationsvorgang zur Bestimmung der momentanen Sehachse, damit der Projektionsvorgang augenzentriert durchgeführt werden kann.

### Figur 5

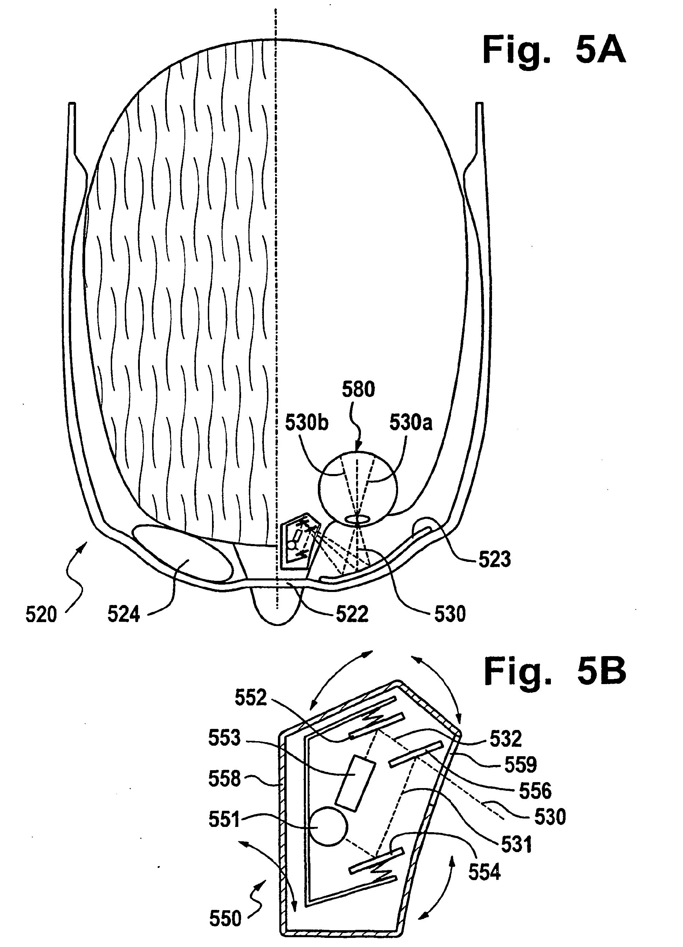

Figur 5A zeigt eine interaktive Brille 520 gemäß einem ersten bevorzugten Ausführungsbeispiel, bei der eine kombinierte Signalerfassungs- und Projektionsvorrichtung 550 im Bereich des Nasenstegs 522 an die Brille 520 angebracht ist. Gemäß der Detailzeichung 5B umfaßt die kombinierte Signalerfassungs- und Projektionsvorrichtung 550 sowohl eine Projektionsvorrichtung 553 als auch eine Signalerfassungsvorrichtung, die zusammen in einem schützenden Gehäuse 558 untergebracht sind. Durch ein lichtdurchlässiges Fenster 559 in einer Außenwand des Gehäuses 558 gelingen Lichtstrahlen 530 in das Innere des Gehäuses 558 und umgekehrt. Das Abschließen des Gehäuses 558 durch das Fenster 559 verhindert jedoch, daß Staub, Schweiß und andere Fremdstöffe den Betrieb der kombinierte Signalerfassungs- und Projektionsvorrichtung 550 stört.

Analog den beschriebenen Systemen gemäß Fig. 3 und 4 werden Lichtstrahlen 530, 530a, 530b erfaßt bzw. projiziert. Die interaktive Brille 520 läßt sich jedoch in ihrem Aufbau dadurch vereinfachen, daß die im Stand der Technik getrennten Spiegel 352 bzw. 452 zur vertikalen bzw. horizontalen Ablenkung des jeweiligen Lichtstrahls 331 bzw. 432 durch einen Taumelspiegel 552 bzw. 554 ersetzt wird. Zwecks einer kompakten Bauweise kann ein teildurchlässiger Spiegel 556 dazu dienen, separate Strahlengänge innerhalb des Gehäuses 558 für das durch das Fenster 559 fallende bzw. projizierte Licht 530 zu ermöglichen. Bevorzugt wird die Innenseite des Brillenglases mit einer für aus dieser Richtung einfallenden Strahlen stark reflektierenden Oberfläche 523 versehen, die als Spiegel für den Strahlengang zwischen dem Auge 580 und dem kombinierten Signalerfassungs- und Projektionsvorrichtung 550 verwendet. Dies trägt zu einer Reduzierung der notwendigen Komponenten bei und führt in der abgebildeten Ausführungsform zu einem vereinfachten, lichtstarken Strahlengang 530, bei dem der Lichtstrahl 530 zwischen Auge 580 und Projektions- bzw. Signalerfassungsvorrichtung 553 bzw. 551 lediglich dreimal reflektiert wird. Wie oben beschrieben, ergibt sich jedoch hieraus eine Einschränkung der Gestaltungsmöglichkeiten der Brille 520.

Die für eine Taumelbewegung des Spiegels 552, 554 notwendige Bewegungsfreiheit läßt sich beispielsweise durch eine kardanische oder federnde Aufhängung des Spiegels 552, 554 erreichen. Mögliche Ausführungsarten eines derartigen Taumelspiegels sind dem Fachmann beispielsweise aus dem Gebiet der Mikrotechnik bekannt. Weitere Lösungen des vorliegenden Ablenkungsproblems, bei der der jeweilige Lichtstrahl 530 auf der Basis elektrochromer, holographischer, elektroholographischer oder sonstiger Lichtbrechungs- oder Lichtreflektionsmechanismen gelenkt wird, sind ohne weiteres denkbar und ebenfalls anwendbar.

Obwohl die interaktive Brille 520 in einer minimalistischen Ausführungsform gezeigt ist, bei der eine kombinierte Signalerfassungs- und Projektionsvorrichtung 550 lediglich für das linke Auge 580 vorgesehen ist, ist es selbstverständlich, daß eine spiegelverkehrt gebaute, zweite kombinierte Signalerfassungs- und Projektionsvorrichtung 550 im Bereich der rechten Hälfte des Nasenstegs 522 für das nicht dargestellte rechte Auge bei Bedarf vorgesehen werden kann.

### Figur 6

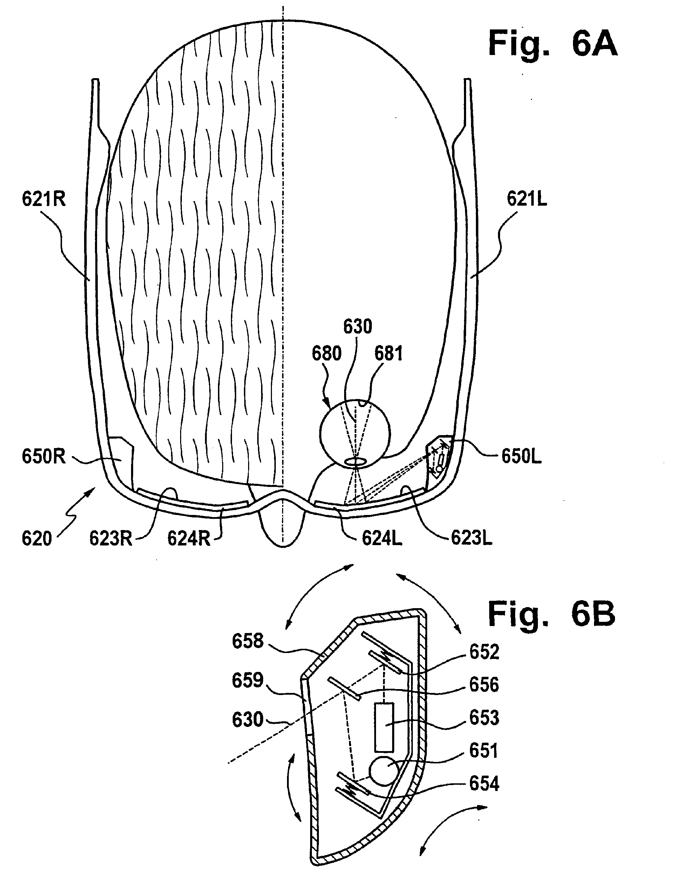

Figur 6A zeigt in Form einer Abänderung der in den Figuren 5A und 5B dargestellten Brille 520 eine interaktive Brille 620 gemäß einem zweiten bevorzugten Ausführungsbeispiel, bei der die linke kombinierte Signalerfassungs- und Projektionsvorrichtungen 650L in dem zwischen dem linken Brillenglas 624L und dem linken Bügelteil 621L liegenden Bereich und die rechte kombinierte Signalerfassungs- und Projektionsvorrichtungen 650R in dem zwischen dem rechten Brillenglas 624R und dem linken Bügelteil 621R liegenden Bereich angeordnet sind.

Eine solche Anordnung der kombinierte Signalerfassungs- und Projektionsvorrichtungen 650L, 650R gegenüber den jeweiligen Brillengläsern 624L, 624R und den jeweiligen Augen 680 ist normalerweise mit der Notwendigkeit verbunden, entweder mehrere Spiegel entlang des Strahlengangs 630 vorzusehen (vgl. Spiegel 322 und 323 in Figur 3) oder dem jeweiligen Brillenglas 624L, 624R eine besondere Form zu verleihen, um eine Erfassung aller Bereiche der Netzhaut 681 zu gewährleisten. Dies schränkt jedoch die Gestaltungsmöglichkeiten der Brille 620 erheblich ein. Um dieses Problem zu umgehen, sieht die interaktive Brille 620 gemäß Figur 6 Brillengläser 624L, 624R vor, deren Innenseite mit einer jeweiligen holographischen Beschichtung 623L, 623R versehen sind. Solche holographischen Beschichtung 623 sind in der Lage, eine beliebige Reflektionstopologie zu emulieren. Zum Beispiel kann eine holographisch beschichtete, flache Oberfläche wie eine sphärisch gekrümmte Oberfläche wirken. Ebenso kann eine holographisch beschichtete, sphärisch gekrümmte Oberfläche wie eine flache Oberfläche wirken. Die Änderung der effektiven Reflektionstopologie hängt lediglich vom holographischen Inhalt der Beschichtung ab. Gemäß der Abbildung sind die holographischen Beschichtungen 623L und 623R spiegelsymmetrisch zueinander ausgebildet und angeordnet.

Figur 6B enthält eine Detailzeichnung der kombinierte Signalerfassungs- und Projektionsvorrichtungen 650L. Analog der in der Figur 5B dargestellten kombinierten Signalerfassungs- und Projektionsvorrichtung 550 umfaßt sie ein Gehäuse 658, eine Projektionsvorrichtung 653 und eine Signalerfassungsvorrichtung 651, jeweilige Taumelspiegel 652 und 654, einen teildurchlässigen Spiegel 656 und ein Gehäusefenster 659.

### Figur 7

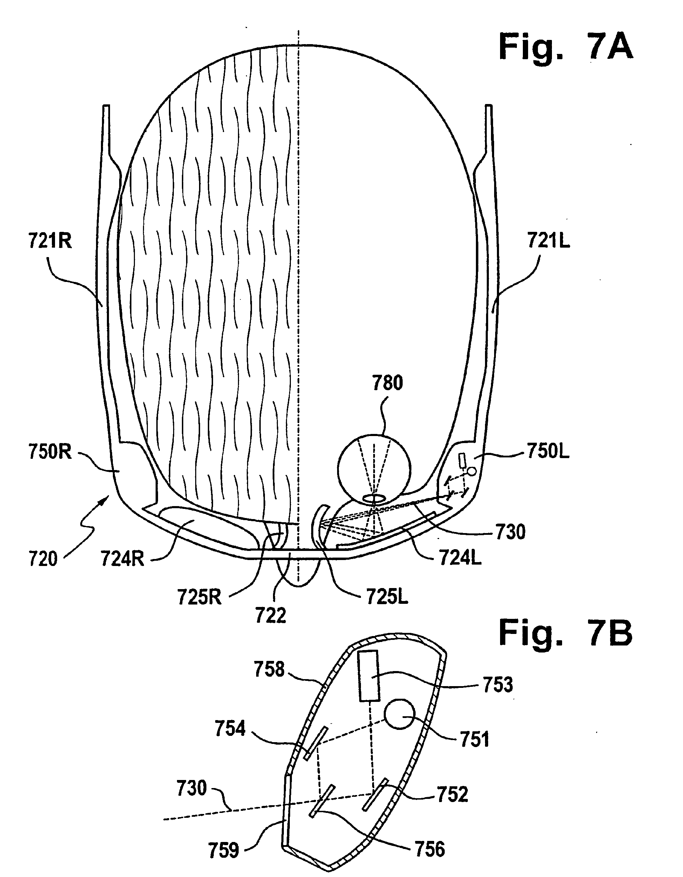

Ähnlich den Figuren 6A und 6B zeigt Figur 7A in Form einer Abänderung der in den Figuren 5A und 5B dargestellten Brille 520 eine interaktive Brille 720 gemäß einem dritten bevorzugten Ausführungsbeispiel, bei der die linke kombinierte Signalerfassungs- und Projektionsvorrichtungen 750L in dem zwischen dem linken Brillenglas 724L und dem linken Bügelteil 721L liegenden Bereich und die rechte kombinierte Signalerfassungs- und Projektionsvorrichtungen 750R in dem zwischen dem rechten Brillenglas 724R und dem linken Bügelteil 721R liegenden Bereich angeordnet sind.

Figur 7B enthält eine Detailzeichnung der kombinierte Signalerfassungs- und Projektionsvorrichtungen 750L. Analog der in der Figur 5B dargestellten kombinierten Signalerfassungs- und Projektionsvorrichtung 550 umfaßt sie ein Gehäuse 758, eine Projektionsvorrichtung 753 und eine Signalerfassungsvorrichtung 751, jeweilige Taumelspiegel 752 und 754, einen teildurchlässigen Spiegel 756 und ein Gehäusefenster 759.

Das oben angesprochene Problem des Strahlengangs 730 wird bei diesem Ausführungsbeispiel durch besonders ausgestaltete Pads 725L und 725R platzsparend gelöst. Typischerweise werden Brillen 720 entweder durch den Nasensteg 722 oder durch sogenannte Pads 725 auf der Nasenwurzel gestützt. In ihrem handelsüblichen Gestalt sind Pads relativ flach, leicht gekrümmt und oval. Zudem sind sie entweder schwenkbar oder taumelnd an einem vom Nasensteg 722 ausgehenden Vorsprung gelagert, um ein angenehmes Anliegen der Pads an die Seitenflächen der Nasenwurzel zu gewährleisten. Im abgebildeten Ausführungsbeispiel sind die Pads 725 als formfeste, längliche Einheiten ausgebildet, die im Bereich des Nasenstegs 722 von der Brille 720 in Richtung Auge 780 herausragen. Auf ihrer jeweiligen, der Nase zugewandten länglichen Seite bilden die Pads 725 die sich auf die Nasenwurzel stützende Aufliegefläche. In ihrem der Brille 720 gegenüber liegenden Endbereich weisen die Pads 725 auf der jeweilig dem Auge zugewandten Seite eine Tragfläche auf, die mit einem Spiegel oder einer spiegelnden Beschichtung, beispielsweise einer Metallbeschichtung oder einer holographischen Beschichtung, versehen ist.

Obwohl das Gestell der Brille 720, einschließlich die Pads 725, eine im Prinzip feste Form aufweist, treten sowohl quasistatische, z.B. durch Materialermüdung und/oder Temperaturänderungen, als auch dynamische Verformungen des Gestells auf. Insbesondere beim Aufsetzen der Brille 720 und bei erschütterungsreichen Aktivitäten ergeben sich Veränderungen der relativen Anordnung der jeweiligen Brillenkomponenten zueinander. Auch ist die relative Anordnung der Brille 720 gegenüber dem Auge 780 keine Konstante. Demgemäß muß sowohl das optische System der Brille 720, d.h. diejenigen Systemkomponenten, die zur optischen Signalerfassung bzw. zur optischen Projektion beitragen, als auch ein eventuell daran angeschlossenes Verarbeitungssystem derart konzipiert und ausgelegt sein, daß solche Anordnungsveränderungen berücksichtigt und/oder kompensiert werden können bzw. keine außerordentlichen Betriebsstörungen verursachen. Dies gilt für alle Arten von interaktiven Brillensystemen.

Gemäß der vorliegenden Offenbarung läßt sich das zuvor angesprochene Problem insbesondere durch eine geeignete Signalverarbeitung der erfaßten und der zu erzeugenden Signale bewältigen. Es können auch fest am Brillengestell in der Nahe des üblichen Strahl engangs 730 angebrachte optische Markierung von der Signalerfassungsvorrichtung 751 zwecks Eichung ihres optischen Systems regelmäßig oder bei Bedarf mit erfaßt werden.

### Figur 8

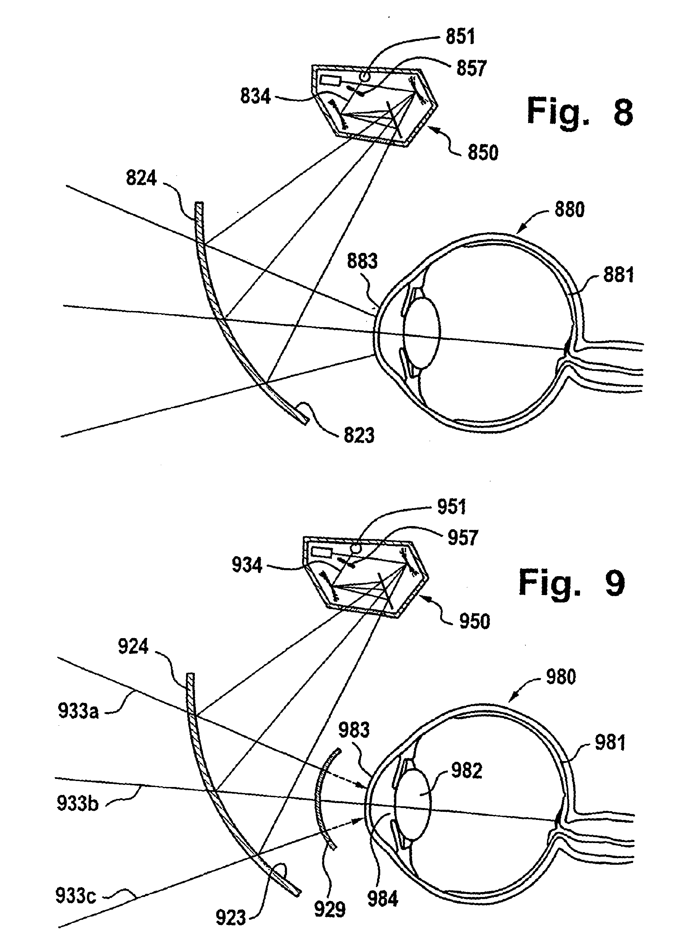

Figur 8 zeigt in Form einer Abänderung der in den Figuren 5A und 5B dargestellten Brille 520 eine interaktive Brille gemäß einem vierten bevorzugten Ausführungsbeispiel, bei der die Signalerfassungsvorrichtung 851 der kombinierten Signalerfassungs- und Projektionsvorrichtungen 850 in der Lage ist, das Hornhautreflexbild mindestens partiell zu erfassen.

Die Hornhaut ist normalerweise rotationssymmetrisch zur Sehachse ausgebildet. Strahlen, die senkrecht auf einen zentralen Bereich der Hornhaut fallen, sind somit konfokal zum optischen System des Auges 880 und bilden die Basis des von der Netzheut 881 tatsächlich wahrgenommenen Bildes. Zudem besteht die Hornhaut 883 zum größten Teil aus Wasser und weist aus diesem Grunde einen sehr hohen Reflektionsgrad bei einer Wellenlänge von ca. 1,1 µm auf. Da diese Wellenlänge im infraroten Spektralbereich liegt, eignet sich eine Erfassung des Hornhautreflexbildes vorwiegend für Infrarotanwendungen, beispielsweise bei Nachtsichtgeräten. Allerdings finden Reflektionen nicht nur an der äußeren, konkaven Hornhautoberfläche, sondern auch im Inneren der Hornhaut statt. Zudem bewirkt die Hornhaut 883 aufgrund ihrer Struktur keine spiegelartige, sondern eine diffuse Reflektion, die mit zunehmender Tiefe des Reflektionsereignisses im Inneren der Hornhaut diffuser wird.

Um ein sinnvolles Hornhautreflexbild zu erhalten, werden im abgebildeten Ausführungsbeispiel effektiv nur diejenigen Strahlen, die senkrecht auf einen zentralen Bereich der Hornhaut fallen, erfaßt. Dies wird durch mehrere Maßnahmen erreicht. Erstens weist das vor dem Auge gelagerte Brillenglas 824, dessen dem Auge 880 zugewandte Seite mit einer für aus dieser Richtung einfallenden Strahlen stark reflektierenden Oberfläche 823 versehen ist, eine besonders gestaltete Form auf, die das senkrecht von der Hornhaut reflektierte Licht derart bündelt, daß es als beinah parallel verlaufende Lichtstrahlen 834 auf die Signalerfassungsvorrichtung 851 fällt, während nicht senkrecht von der Hornhaut reflektiertes Licht in eine andere Richtung gelenkt wird. Alternative kann das Brillenglas 824 andersartig gestaltet sein, jedoch eine teilsdurchlässige holographisch reflektierende Schicht 823 aufweisen, die ebenfalls eine derartige Bündelung des senkrecht von der Hornhaut reflektierten Lichtes bewirkt, daß es als beinah parallel verlaufende Lichtstrahlen 834 auf die Signalerfassungsvorrichtung 851 fällt, während nicht senkrecht von der Hornhaut reflektiertes Licht in eine andere Richtung gelenkt wird. Zweitens wird eine Blende 857 kurz vor der Signalerfassungsvorrichtung 851 vorgesehen, die eine Erfassung derjenigen Lichtstrahlen verhindert, deren Einfallswinkel außerhalb einem engen Einfallswinkelbereich der wie oben beschriebenen, beinah parallel verlaufenden Lichtstrahlen 834 liegt.

### Figur 9

Figur 9 zeigt in Form einer Abänderung der in den Figuren 5A und 5B dargestellten Brille 520 eine interaktive Brille gemäß einem fünften bevorzugten Ausführungsbeispiel, bei der ein sphärisches oder sphärisch wirkendes teildurchlässiges spiegelndes Zusatzelement 929 zwischen dem Brillenglas 924 und dem Auge 980 angeordnet ist. Bevorzugt ist das Zusatzelement 929 konfokal zum optischen System des Auges 980 angeordnet.

Der Reflektionsgrad eines solchen Zusatzelements 929 läßt sich an die Bedürfnisse des Informationssystems anpassen. Es kann zwischen einem hohen Reflektionsgrad, was eine sehr gute Erfassung auf das Auge 980 gerichteter Lichtstrahlen 933a-933c ermöglicht, und einem niedrigen Reflektionsgrad, was eine Beeinträchtigung der durch das Auge 980 erfolgenden Wahrnehmung vermeidet, gewählt werden. Bevorzugt weist das Zusatzelement 929 einen niedrigen (beispielsweise unter 10%), über seine gesamte Reflektionsfläche homogen Reflektionsgrad auf. Dahingegen weisen reflektierende Organe des Auges 980, zum Beispiel die Kornea 983 oder die Retina 981, zum Teil sehr starke örtliche Reflektionsabhängigkeiten. Ähnliche Aussagen betreffen die spektralen Reflektionsabhängigkeiten des Zusatzelements bzw. der reflektierenden Organe des Auges 980. Während das Zusatzelement 929 bevorzugt derart ausgebildet werden kann, daß es einen homogenen Reflektionsgrad über alle relevanten Spektralbereiche aufweist, weisen die verschiedene Organe des Auges 980 sehr unterschiedliche Absorbtionsgrade auf, die in vielen Fällen auch starke örtliche Schwankungen unterworfen sind.

Außer der Teilreflektion soll das Zusatzelement 929 möglichst keine Auswirkung auf das darauf fallende Licht ausüben. Aus diesem Grund wird das Zusatzelement 929 bevorzugt aus einem homogenen lichtdurchlässigen und ungefärbten Material und mit einer in Richtung der auf den Augenmittelpunkt gerichteten Lichtstrahlen konstanten Dicke gefertigt. Durch das Aufbringen einer Antireflexbeschichtung auf der dem Auge 980 zugewandten Seite des Zusatzelements 929 läßt sich eine verbesserte Lichtdurchlässigkeit erzielen.

Die reflektierende Kontur eines solchen Zusatzelements 929 ist wohl definiert, und kann dem Informationssystem demgemäß als bekannte Information zur Verfügung gestellt werden, während die Kontur der relevanten reflektierenden Organe des Auges 980 erst ermittelt werden muß. Letzteres kann mit zum Teil nicht unerheblichem Aufwand verbunden sein. Die Erfassung auf das Auge 980 gerichteter Lichtstrahlen 933a-933c über ein solches Zusatzelement 929 kann somit hochwertige Bilder des Blickfeldes liefern.

Im abgebildeten Ausführungsbeispiel werden effektiv nur diejenigen Strahlen, die senkrecht auf das Zusatzelement 929 fallen, erfaßt. Dies wird durch die folgenden Maßnahmen erreicht:

Aufgrund der teilsreflektierenden Oberfläche des Zusatzelements 929 wird ein entsprechender Teil derjenigen Strahlen 933a-933c, die senkrecht auf die Oberfläche des Zusatzelements 929 fallen, senkrecht zurückreflektiert, während andere Strahlen von der Oberfläche des Zusatzelements 929 gemäß dem Reflektionsgesetz "Einfallswinkel gleich Reflektionswinkel" entsprechend schräg zurückreflektiert werden. Die senkrecht zur Oberfläche des Zusatzelements 929 zurückreflektierten Lichtstrahlen legen den gleichen Weg zurück, den sie gekommen sind, und treffern somit auf das dem Auge vorgelagerte Brillenglas 924. Die dem Auge 980 zugewandte Seite des Brillenglases 924 ist mit einer für aus dieser Richtung einfallenden Strahlen stark reflektierenden Oberfläche 923 versehen, und weist eine besonders gestaltete Form oder eine besonderes ausgebildete Beschichtung auf, die die senkrecht vom Zusatzelement reflektierten Lichtstrahlen derart bündelt, daß sie als beinah parallel verlaufende Lichtstrahlen 934 auf die Signalerfassungsvorrichtung 951 fallen, während nicht senkrecht vom Zusatzelement reflektierte Lichtstrahlen in eine andere Richtung gelenkt werden. Desweiteren wird eine Blende 957 kurz vor der Signalerfassungsvorrichtung 951 vorgesehen, die eine Erfassung derjenigen Lichtstrahlen verhindert, deren Einfallswinkel außerhalb einem engen Einfallswinkelbereich der wie oben beschriebenen, beinah parallel verlaufenden Lichtstrahlen 934 liegt.

Soll das über das Zusatzelement 929 erfaßte Bild des Gesichtsfeldes die Grundlage für eine mit dem tatsächlich wahrgenommenen Gesichtsfeld korrelierte Projektionen, so muß die Korrelation zwischen dem erfaßten Licht und dem wahrgenommenen Gesichtsfeld ermittelt werden. Gemäß dem dargestellten fünften Ausführungsbeispiel wird diese Korrelation durch eine bevorzugte konfokale Anordnung des Zusatzelements 929 zum optischen System des Auges 980 erreicht. Es wird deshalb bevorzugt, daß das Zusatzelement 929 über eine justierbare Aufhängung derart an der Brille befestigt ist, daß sich die Position des Zusatzelements 929 sowohl 1 in vertikaler als auch in zwei horizontalen Richtungen nachjustieren läßt.

Konfokalität ist im grundegenommen dann gegeben, wenn das Zusatzelement 929, optisch gesehen, rotationssymmetrisch zur Sehachse und mit einem Abstand zur Linse 982 angeordnet ist, daß der optische Mittelpunkt des optischen Systems des Auges mit dem Mittelpunkt der durch das sphärische oder sphärisch wirkende Zusatzelement definierten Kugel übereinstimmt. Die Sehachse läßt sich zu diesem Zwecke ausreichend über die Ausrichtung der Pupille 984 bestimmen, die durch ihre scharfe Konturen leicht erkennbar ist, und deren Ausrichtung aufgrund ihrer runden Form licht bestimmbar ist. Zudem ist aufgrund der spärischen oder sphärisch wirkenden Form des Zusatzelements 929 keine Schwenkung des Zusatzelements 929 um die möglichen Schwenkachsen des Auges 980 notwendig, um Konfokalität zu gewährleisten. Denn auch bei einer Verdrehung des Auges bleibt durch eine entsprechende vertikale und/oder horizontale Verschiebung des Zusatzelements 929 zumindest ein wesentlicher Teil des Zusatzelements 929, optisch gesehen, rotationssymmetrisch zur Sehachse. Was den Abstand zur Linse 982 betrifft, gibt es verschiedene Möglichkeiten, den notwendigen Abstand zu bestimmen. Zum Beispiel kann eine optische oder akustische Vermessung der Hornhaut 983 vorgenommen werden, deren Krümmung einen sehr guten Richtwert für die richtige Anordnung des Zusatzelements 929 angibt. Es können auch Netzhaut- oder Hornhautreflexbilder zumindest partiell erfaßt werden, und anhand eines Vergleichs der Reflexbilder mit dem über das Zusatzelement 929 erfaßten Licht der richtige Abstand bestimmt werden.

Aufgrund der sphärischen oder sphärisch wirkenden Realisierung, beispielsweise durch eine holographische Beschichtung, der teilsreflektierenden Oberfläche des Zusatzelements 929 sowie durch diese konfokale Anordnung des Zusatzelements zum Auge 980 sind lediglich diejenigen Strahlen 933a-933c, die senkrecht auf die Oberfläche des Zusatzelements 929 fallen, konfokal zum optischen System des Auges 980 und stimmen somit mit den auf die Netzhaut fallenden Strahlen überein.

### Figur 10

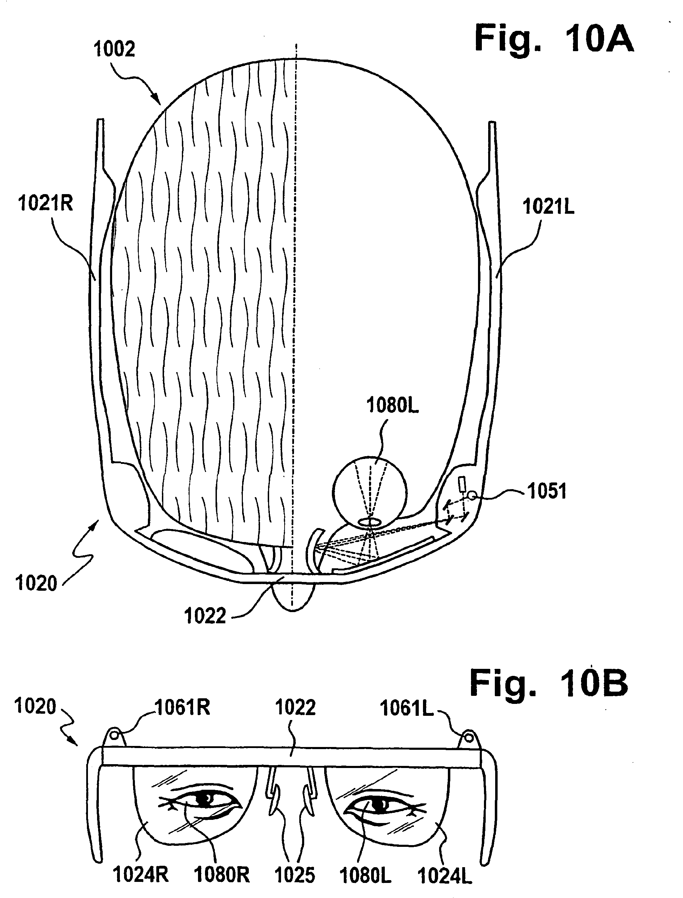

Figur 10 zeigt eine Draufsicht (Fig. 10A) und eine Frontansicht (Fig. 10B) einer Brille 1020 gemäß einem sechsten Ausführungsbeispiel, bei dem zwei Sensorvorrichtung 1061R und 1061L, beispielsweise zwei Festkörper-Kameras, zum Beispiel CCD- oder TTL-Kameras, zwecks weiterer Signalerfassung, insbesondere aus dem sichtbaren Blickfeld, vorgesehen sind. Die Figur 10B zeigt auch das linke und rechte Auge 1080L bzw. 1080R eines möglichen Trägers 1002 der Brille 1020. Der Übersichtlichkeit halber sind jedoch keine anderen Merkmale des Benutzers 1002 in der Figur 10B dargestellt.

Um das Auftreten einer Parallaxe zwischen den von der jeweiligen Kamera 1061R, 1061L und dem ihr zugeordneten Auge erfaßten bzw. wahrgenommenen Bildern zu vermeiden, sollen die Kameras 1061 den Augen bezüglich ihrer "Sehachsen" möglichst achsengleich angeordnet sein. In Anbetracht der Systemgröße solcher Festkörper-Kameras 1061 hat es sich beim heutigen Stand der technik als sinnvoll erwiesen, die Kameras 1061 wie abgebildet im vorderen Bereich der jeweiligen Bügelteile 1021L, 1021R anzuordnen. Auch eine Montage im Bereich des Nasenstegs 1022, z.B. in den Pads 1025, ist sinnvoll. Nach einer weiteren Miniaturisierung werden die Festkörper-Kameras 1061 im Brillengestell über den jeweiligen Brillengläser 1024L, 1024R angeordnet werden können, um eine weitere Achsengleichheit zu erreichen. Es ist absehbar, daß Festkörper- oder andersartige Lichterfassungssysteme in der Zukunft in das Brillenglas 1024, das selbstverständlich auch ein Kunststoff oder sonstiger lichtdurchlässiger Stoff sein kann, werden eingebaut werden können. Eine solche Anordnung der Kameras 1061 würde eine mit dem jeweiligen Auge 1080L, 1080R achsengleiche, beinah konfokale Signalerfassung ermöglichen.

Bei einer achsenungleichen Anordnung der Sensorvorrichtungen 1061 zu den jeweiligen Augen 1080L, 1080R sollen die aus den Sensorvorrichtungen 1061 gewonnenen Informationen gegebenenfalls in Korrelation mit den Augen 1080 gebracht werden. Eine solche Korrelation ist insbesondere dann wichtig, wenn die Sensorvorrichtungen 1061 durch Kameras 1061 realiziert werden, und ein Überlagerungsbild anhand aus den Kameras 1061 gewonnener Bildinformationen in das jeweilige Auge 1080L, 1080R projiziert werden sollen.

Wird das von den Kameras 1061 aufgenommene Bild einfach auf das jeweilige Auge 1080L, 1080R projiziert, so kommt es zur sogenannten Parallaxe, bei der das "Gesichtsfeld" der jeweiligen Kamera 1061L, 1061R nicht mit dem natürlich wahrgenommenen Gesichtsfeld übereinstimmt. Insbesondere bei einer von der Ruhestellung abweichenden Verdrehung der Augen 1080 oder bei im näheren Gesichtsfeld liegenden Gegenständen würde die Parallaxe bei einer Überlagerung zu einer abnormalen Wahrnehmung führen. Denn in solchen Fällen läge die Sehachse des Auges 1080 schräg zur "Sehachse" der jeweiligen Kamera 1061L, 1061R.

Bei der Korrelation in diesem Sinne wird nur der Teil des von den Kameras 1061 erfaßten Bildes in das jeweilige Auge 1080L, 1080R projiziert, der in entsprechender "Korrelation" zur Sehachse des jeweiligen Auges 1080L, 1080R liegt. Im einfachsten Fall wird durch die Signalerfassungsvorrichtung 1051 ein zumindest partielles Reflexbild des Gesichtsfeldes vom jeweiligen Auge 1080L, 1080R erfaßt. Kennzeichnende Bildpunkte, die sowohl im erfaßten Reflexbild als auch in den von den Kameras 1061 erfaßten Bilder aufzufinden sind, dienen dann als Referenzpunkte für eine perspektivisch richtige Projektion der von den Kameras 1061 erfaßten Bildinformationen auf das Auge 1080. Ähnlich können die aus dem Auge 1080 erfaßten Signale dazu dienen, die Blickrichtung des jeweiligen Auges 1080L, 1080R bezüglich dem Koordinatensystem der Brille 1020 zu bestimmen, um aus diesen Winkelinformationen eine mathematisch basierte Korrelation zu durchführen.

Allerdings ist eine Korrelation auch bei Systemanwendungen sinnvoll, bei denen die Augen 1080 an der Wahrnehmung des Gesichtsfeldes verhindert werden. Dies ist beispielsweise bei der Anwendung einer geschlossenen, sogenannten "virtual reality" Brille 1020 (wie abgebildet, allerdings mit lichtundurchlässigen Gläsern 1024) der Fall, bei der den Augen 1080 lediglich ein künstlich erzeugtes Bild präsentiert wird. Im einem solchen Fall könnte die besprochene Korrelation zum Beispiel darin bestehen, daß die Blickrichtung des Auges 1080 wie oben beschrieben erfaßt wird, und daß ein der Orientierung des jeweiligen Auges 1080L, 1080R entsprechendes, virtuell erzeugtes Bild hineinprojiziert wird. Allerdings dient hier die Brille 1020 als Koordinatensystem. Wird jedoch noch die Lage und Orientierung der Brille 1020, beispielsweise anhand der von der Kameras 1061 erfaßten Bildern, ermittelt, so kann eine Korrelation zwischen dem jeweiligen Auge 1080L, 1080R und der Umgebung hergestellt werden. Ein solches System ließe sich beispielsweise in einem virtuellen Erlebnishaus, ähnlich einem Geisterhaus, anwenden. Jedem, der gerade auf einem Laufbahn geht, könnte zum Beispiel ein virtuelles Bild in die Augen projiziert werden, das ihm das Gefühl verleiht, er liefe auf schwimmenden Baumstämmen inmitten eines wilden Flüsses.

Es soll an dieser Stelle hervorgehoben werden, dass das vorstehend anhand der Figuren 5 bis 10 beschriebene Informationssystem nicht unbedingt mit einer kombinierten Signalerfassungs- und Projektionsvorrichtung arbeiten muß. Es ist gleichermaßen möglich, mit einer Ausführung des Systems zu arbeiten, bei der die Signalerfassungsvorrichtung von der Projektionsvorrichtung getrennt ist bzw. bei dem eine der beiden Vorrichtungen fehlt.

### Figur 11

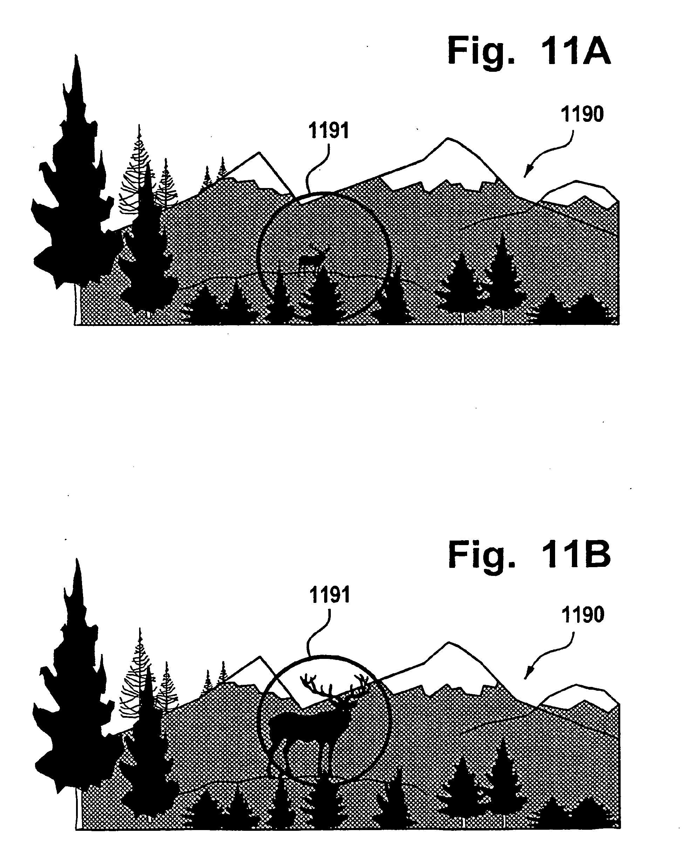

Gemäß einem siebten Ausführungsbeispiel umfaßt das Informationssystem Mittel, die das Bereitstellen einer Fernglasfunktion ermöglichen. Die Figuren 11A und 11B stellt den wahrnehmbaren Effekt der Fernglasfunktion an einen Benutzer dar. Figur 11A zeigt das natürlich wahrgenommene Gesichtsfeld 1190 eines Benutzers eines gemäß dem sieben Ausführungsbeispiel gestalteten Informationssystems. Obwohl das Gesichtfeld 1190 horizontal ca. 170° und vertikal ca. 110° der Umgebung einschließt, bildet lediglich ein kleiner Bereich 1191 von wenigen Grad um die Sehachse herum den Bereich des schärfsten Sehens 1191.

Durch seine Erfassung von Licht aus dem Gesichtsfeld und die zuvor beschriebene Möglichkeit einer Projektion von Bildinformationen in das Auge kann das Informationssystem derart ausgestaltet werden, daß dieser Bereich 1191 beispielsweise auf Knopfdruck nach entsprechender Bearbeitung der erfaßten Bildpunkte mittels einer von der Informationsvorrichtung umfaßten Auswertevorrchtung optisch vergrößert auf den Bereich des schärfsten Sehens 1191 projiziert wird. Wie zuvor beschrieben, kann der Grad der Wahrnehmung eines so projizierten Bildes im Verhältnis zum natürlich wahrgenommenen Bild durch die Helligkeit der projizierten Bildpunkte gesteuert werden. Wird das Gesichtsfeldlicht beispielsweise als Reflexbild aus dem Auge erfaßt, so gewährleistet eine räumliche oder zeitliche Trennung der Erfassung und der Projektion, daß die Projektion die Erfassung nicht beeinflußt.

Bei einem handelsüblichen Fernglas geht dadurch, daß das gesamte Gesichtsfeld vergrößert dargestellt wird, den räumlichen Bezug zur Umgebung verloren. Als Konsequenz ist eine durch ein Fernglas schauende Person nicht in der Lage, sich gleichzeitig dabei fortzubewegen. Dieses Phenomen ist wohl bekannt.

Dadurch, daß das Informationssystem gemäß der vorliegenden Offenbarung durch seine Erfassung von Signalen aus dem Auge die Sehachse bzw. die Position der Fovea centralis relativ zum optischen System der Brille ermitteln kann, ist das Informationssystem in der Lage, diesen Nachteil eines handelsüblichen Fernglases zu vermeiden. Zum Beispiel kann die Projektion auf eine wie in Figur 11B dargestellte Art erfolgen, bei der lediglich ein kleiner, im natürlichen Gesichtsfeld unmittelbar um die Sehachse liegender Bereich 1191 vergrößert auf die Fovea centralis projiziert wird, während keine projizierten Bildinformationen dem restlichen Gesichtsfeld überlagert werden. Somit bleibt die vom Benutzer peripher wahrgenommene Szene trotz teleskopischer Darbietung des relevantesten Bereichs des Gesichtsfeldes gleich. Um diesen Effekt zu erzielen, muß die Helligkeit der in das Auge hineinprojizierten Bildinformationen selbstverständlich so gewählt werden, daß das gewünschte Wahrnehmungsverhältnis zwischen dem natürlichen und dem projizierten Bild entsteht. Dieses System hat auch den Vorteil. daß der für die Vergrößerung bildverarbeitungsmäßig notwendige Aufwand in Grenzen gehalten wird, denn es wird nur ein ausgewählter Bildbereich 1191 des Gesichtsfeldes 1190 bearbeitet.

Gemäß einer nicht dargestellten, eleganten Ausführungsform wird ein Vergrößerungsbild derart in das Auge hineinprojiziert, daß das projizierte Bild in einem ringförmigen Grenzbereich zwischen dem Bereich des schärfsten Sehens 1191 und dem restlichen Bereich der Netzhaut mit zunehmender Nähe zur Sehachse stärker vergrößert wird. Dabei wird am äußeren Rande gar nicht vergrößert und am inneren Rande mit dem gleichen "Zoomfaktor" vergrößert, wie das in das Innere des Rings, d.h. auf die Fovea centralis, projizierte Vergrößerungsbild. Bei entsprechend gewählter Helligkeit der projizierten Bildinformationen entsteht somit ein weicher Übergang zwischen der peripheren Szene und dem teleskopisch Gesehenen.

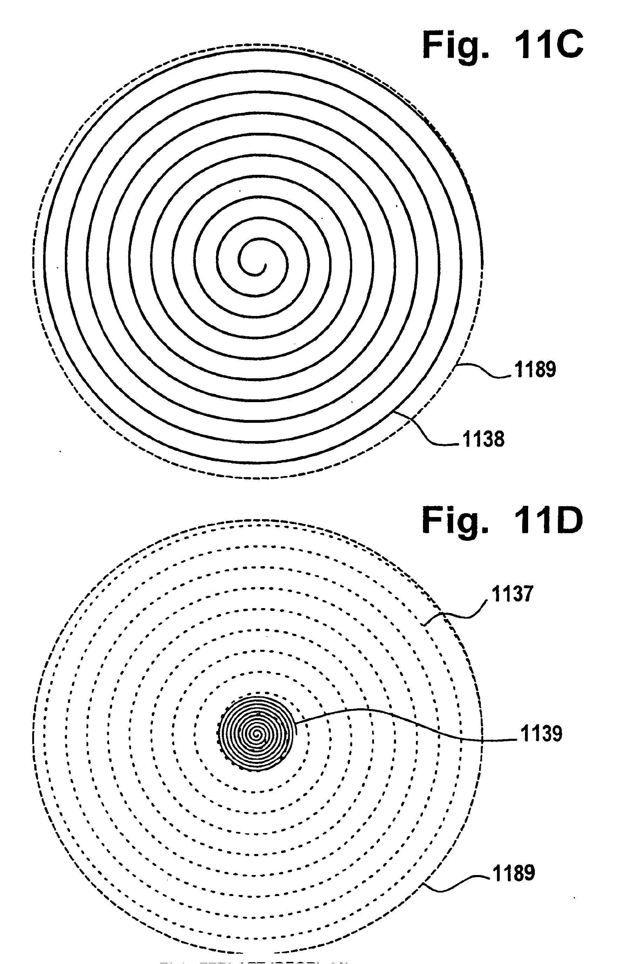

Die Figuren 11C und 11D stellen schemenhaft dar, wie eine Vergrößerung des auf die Fovea centralis natürlich fallenden Bildes durch eine Abänderung eines Abtastmusters 1138, 1139 bei der Abtastung eines Reflexbildes erreicht werden kann. Obwohl Projektionsmuster 1137 und Abtastmuster 1138, 1139 in den Figuren 11C und 11D der Erläuterung halber in einer gemeinsamen Ebene dargestellt sind, kann es bei dem Informationssystem gemäß der vorliegenden Offenbarung durchaus sein, daß auf die Netzhaut projiziert wird, während die Abtastung beispielsweise von der Hornhaut erfolgt.

Figur 11C stellt ein typisches Abtastmuster 1138 schematisch dar, das das das Gesichtsfeld reflektierende Gebiet 1189 der Hornhaut oder Netzhaut abtastet. Bei diesem stark vereinfachten Beispiel wird der Verständlichkeit halber davon ausgegangen, daß die jeweiligen Bildpunkte des sequentiell abgetasteten Bildes nach eventueller bildverarbeitender Aufbereitung ihrer Reihenfolge nach als korrespondierende Bildpunkte des sequentiell in das Auge projizierten Bildes zurückprojiziert werden. Im dargestellten Beispiel stimmt das Abtastmuster 1138 somit mit dem Projektionsmuster 1137 trotz eventueller räumlicher oder zeitlicher Trennung des Abtast- und des Projektionsstrahls überein. Ist eine Vergrößerung einer zentralen Bereichs des Gesichtsfeldes erwünscht, so kann die Abtastung gemäß einem abgeänderten Abtastmuster 1139 erfolgen, das in jenem zentralen Bereich eine Erhöhung der Dichte der abgetasteten Bildpunkte bewirkt. Werden diese mit erhöhter Dichte aufgenommenen Bildpunkte bei der Projektion korrespondierend, jedoch mit geringerer Dichte zurückprojiziert, so ergibt sich ein vergrößertes Bild.

### Figur 12

Gemäß einem achten Ausführungsbeispiel der vorliegenden Offenbarung stellt das Informationssystem ein Führungssystem dar. Zu diesem Zweck umfaßt die Informationsvorrichtung des Informationssysteme Lagesensoren, beispielsweise Beschleunigungsmeßvorrichtungen oder GPS-Empfänger, sowie eine Datenbank oder Datenbankanbindung, die Orientierungsdaten liefert. Eine derartige Datenbank läßt. sich beispielsweise über einen die Daten tragenden CD-ROM, einen DVD oder ein anderes austauschbares Speichermedium in Verbindung mit einem entsprechenden Lesegerät realisieren. Verfahren und Vorrichtungen zur Gewinnung von Ortungsinformationen, die beispielsweise den Standort bestimmen oder deren Bestimmung ermöglichen, durch eine Kombination solcher Orientierungsdaten mit aus den Lagesensoren gewonnenen Daten sind bekannt. In einer typischen Vorrichtung umfassen die Orientierungsdaten Karteninformationen, die in Zusammenhang mit aus den Lagesensoren gelieferten Signalen zur Ortsbestimmung verwendet werden. Die Herstellung einer Korrelation oder einer Abhängigkeit beispielsweise bei der Gewinnung oder der Darstellung solcher Ortungsinformationen zwischen aus dem Auge erfaßten Signalen oder aus dem Gesichtsfeld erfaßten Licht und dem Zurve-rfUgungstellen der Informationen übersteigt das Fachnotorische jedoch bei weitem.

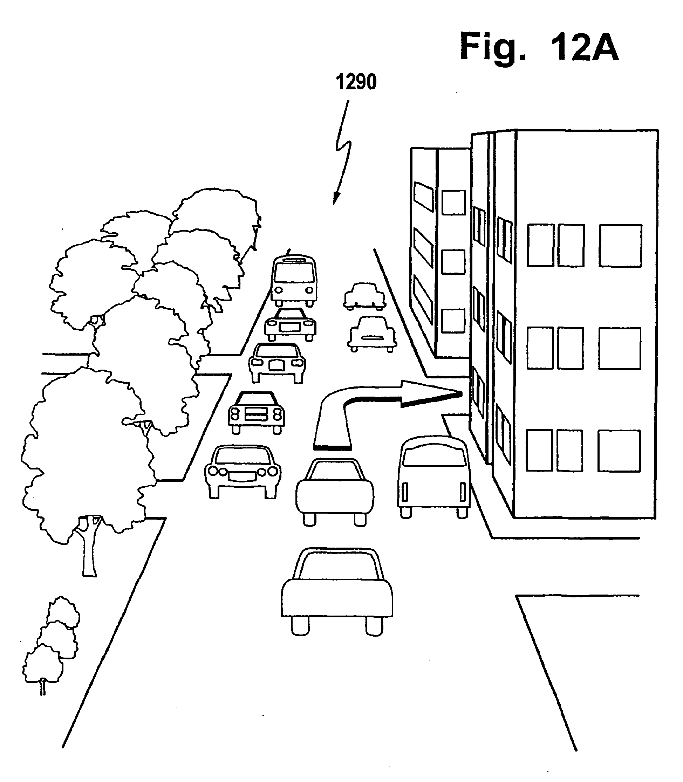

Die Figuren 12A bis 12E zeigen das wahrgenommen Gesichtsfeld 1290 eines Benutzers eines gemäß dem achten Ausführungsbeispiel gestalteten Informationssystems. Bei einem solchen Informations-bzw. Führungsssystem wird das erfaßte Gesichtsfeldlicht in Anbetracht der gewonnenen Ortungsinformationen mittels einer Mustererkennung unter Berücksichtigung der für den ermittelten Aufenthaltort zur Verfügung stehenden Daten ausgewertet. Dabei werden für den ermittelten Aufenthaltort zu erwartende Orientierungshinweise, wie markante Bauten, Seitenstraße, o. ä., erkannt, so daß eine beispielsweise visuelle oder akustische Führung bzw. Identifizierung ggf. erfolgen kann.

Im dargestellten Beispiel gemäß Figur 12A dient das Führungssystem der Navigation. Dabei wir beispielsweise anhand einer berechneten oder vorgegebenen Route, zur Verfügung stehender Karteninformation und des momentanen Aufenthaltortes festgestellt, daß in die übernächste Straße auf der rechten Seiten eingebogen werden soll. Diese Straße wird auf der Basis des erfaßten Gesichtsfeldlichts mittels einer Mustererkennung erkannt, woraufhin ein auf die Straße weisender Hinweispfeil per Projektion unter Berücksichtung der durch das System ermittelten Blickrichtung ortsgetreu in das Gesichtfeld eingeblendet wird. Ähnlich könnte das Führungssystem dem Verkehrsteilnehmer akustische Mitteilungen liefern, beispielsweise "Rechts abbiegen nach 50m" oder "Jetzt rechts".

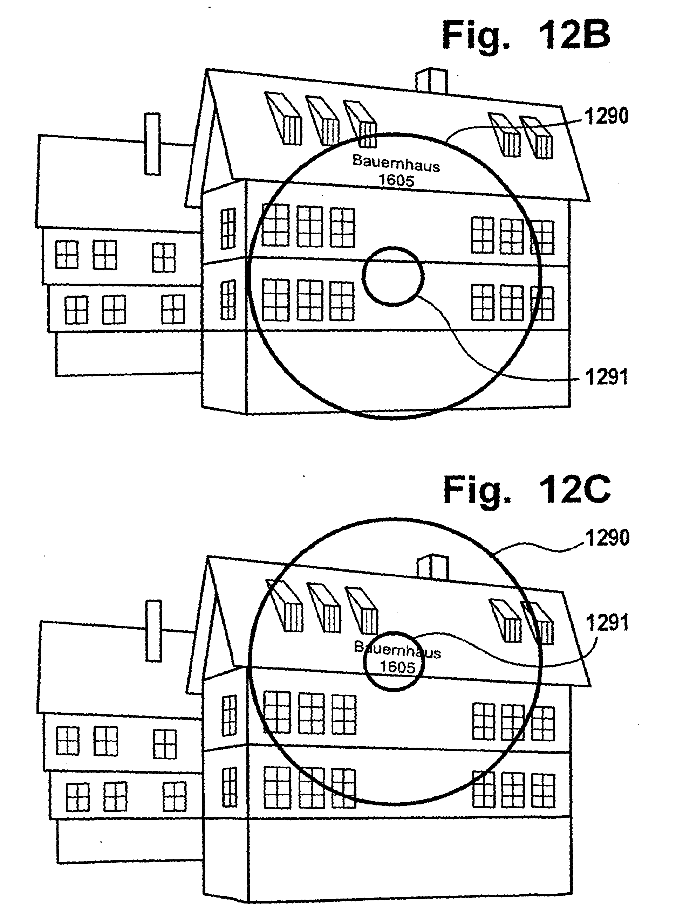

Im in den Figuren 12B und 12C dargestellten Beispiel dient das Führungssystem der Information. Zum Beispiel kann einem Benutzer Information über seine unmittelbare Umgebung wahlweise zur Verfügung gestellt werden. Gemäß Figur 12B schaut ein das Informationssystem benutzender Tourist ein markantes Gebäude an und betätigt eine physikalisch vorhandene oder virtuell in das Gesichtsfeld eingeblendete Aktivierungstaste. Das Gebäude wird anschließend anhand des ermittelten Aufenthaltortes und einer auf das erfaßte Gesichtsfeldlicht basienden Mustererkennung oder eines die Kopfrichtung bestimmenden elektronischen Kompasses bestimmt, woraufhin Informationen zu dem Gebäude zur Verfügung gestellt werden. Diese können aus einer Datenbank oder sonstiger Informationsquelle stammen und ließen sich beispielsweise interaktiv über ein kontextabhängiges Menü, das die zur dem jeweiligen Gebäude zur Auswahl stehenden Informationen visuell oder akustisch auflistet, auswählen. Die Selektion könnte über eine Sprachsteuerung oder durch eine Fixierung mit den Augen erfolgen. Näheres zur augengesteuerten Menüführung wird in einem späteren Abschnitt dieser Beschreibung erläutert.

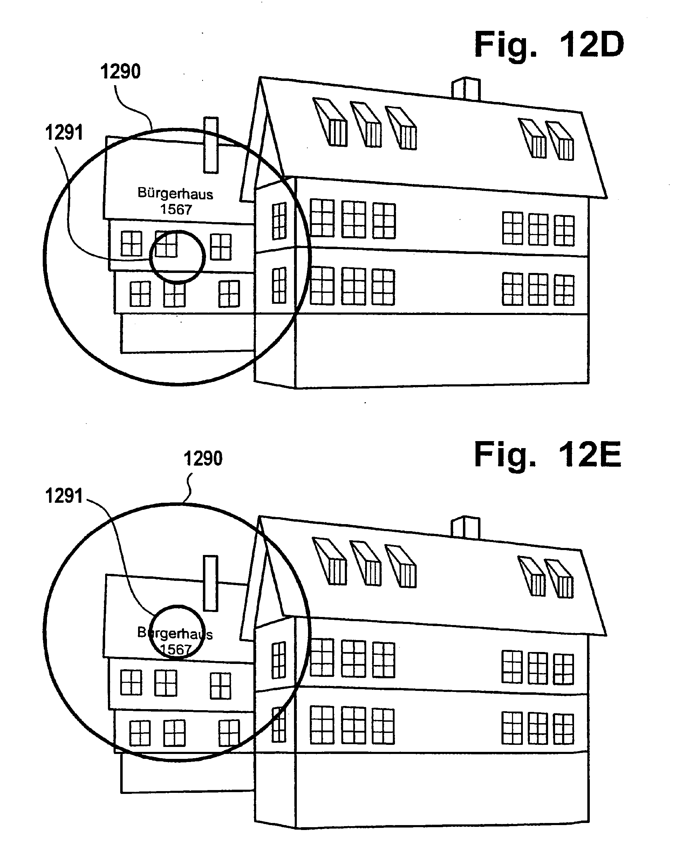

Gemäß Figur 12B werden historische Daten per Projektion in das Gesichtsfeld eingeblendet. Dabei ermittelt das System aus dem erfaßten Gesichtfeldlicht eine geeignete Einblendestelle. beispielsweise vor einem eintönigen Dach oder vor dem Himmel. Entsprechend der Einblendstelle werden die Daten eingeblendet. Typischerweise wird die Fovea centralis vorerst nicht auf die Einblendstelle gerichtet sein, weshalb die eingeblendeten Daten vorerst als unscharfe periphere Erscheinung wahrgenommen werden. Erst durch eine entsprechende Schwenkung der Blickrichtung gemäß Figur 12C werden die ortsfest eingeblendeten Daten auf die Fovea centralis abgebildet. Wird der Blick auf ein anderes vom System erkanntes Gebäude gerichtet, so könnten, sich die eingeblendeten Information gemäß den Figuren 12D und 12E ändern. In den Figuren stellt der Kreis 1290 das wahrgenommene Gesichtsfeld dar, während der Kreis 1291 den von der Fovea centralis erfaßten Bereich des Gesichtsfeldes kennzeichnet.

Durch eine wie in der Figur 1 dargestellte, kompakte und tragbare Bauweise könnte ein solches Orientierungssystem von einem Füßgänger, einem Radfahrer, einem Motorradfahrer oder einem sonstigen Fahrzeugfahrer getragen werden.

### Figur 13

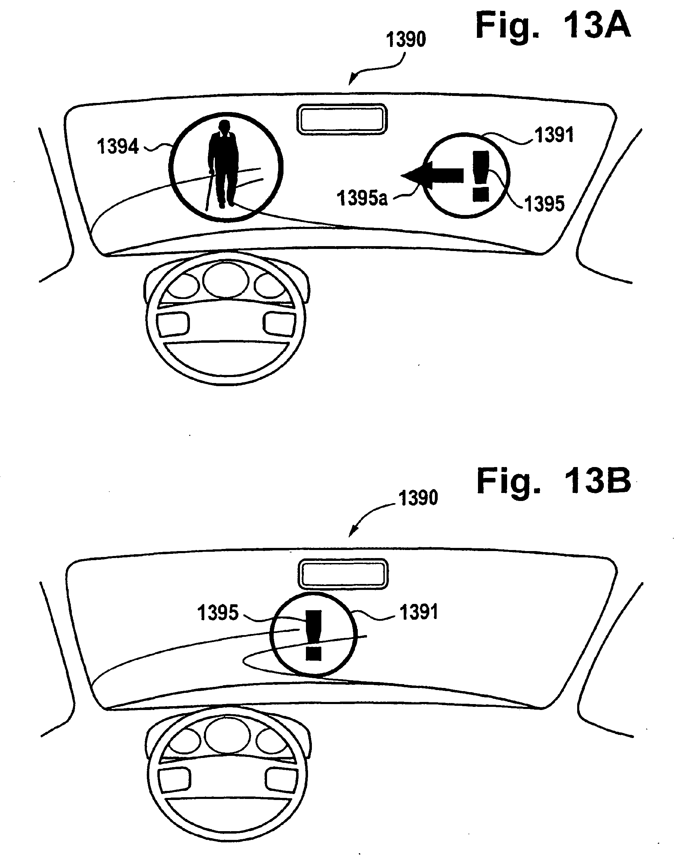

Gemäß einem in den Figuren 13A und 13B dargestellten, neunten Ausführungsbeispiel der vorliegenden Offenbarung fungiert das Informationssystem als Fahrhilfe. Die Figuren zeigen das wahrgenommene Gesichtsfeld 1390 eines Benutzers eines solchen Systems.

Bevorzugt umfaßt die Informationsvorrichtung des Informationssystems einen Abstandssensor, beispielsweise einen optischen oder akustischen Abstandsmeßgerät oder eine Radarvorrichtung, oder ist an ein entsprechendes abstandmeßendes System angeschlossen, das den Abstand zwischen einem Fahrzeug und sich in Fahrtrichtung vor dem Fahrzeug befindlichen Gegenständen ermittelt. Bei einer stereoskopischen Erfassung von Licht aus dem Gesichtsfeld könnte der Abstand mittels einer Paralaxeberechnung ermittelt werden, bei der die Änderung der Position des Gegenstands in einem jeweilig links und rechts erfaßten Bild Auskunft über den Abstand vermittelt.

Wird zum Beispiel über eine ebenfalls von der Informationsvorrichtung umfaßte Auswertevorrichtung festgestellt, daß das Fahrzeug sich auf Kollisionskurse mit dem Gegenstand befindet, so kann beispielsweise ein Warnzeichen 1395 in den Bereich des schärfsten Sehens 1391 und ein Warnkreis 1394 um den gefährlichen Gegenstand mittels einer wie zuvor beschriebenen Projektion eingeblendet werden. Befindet sich der Gegenstand außerhalb oder am Rande des Bereichs des peripheren Sehens, so kann ein weiteres Warnzeichen 1395a darauf hinweisen, wo der Gefahr sich birgt. Dies ist in der Figur 13A dargestellt.

Über Sensoren oder dem erfaßten Gesichtsfeldlicht lassen sich auch andere der Fahrsicherheit relevanten Informationen ermitteln. Zum Beispiel könnte eine Auswertevorrichtung die Fahrbahnmarkierungen einer innerhalb des Gesichtsfeldes liegenden Fahrbahn per Mustererkennung erkennen und daraus die höchstmögliche Geschwindigkeit, insbesondere bei Kurven, berechnen. Stellt das Informationssystem selbstständig oder durch Anbindung an das Instrumentensystem eines Fahrzeug fest, daß das Fahrzeug diese errechnete Höchstgeschwindigkeit überschritten hat, so kann ein Warnzeichen 1395 in den Bereich des schärfsten Sehens 1391 eingeblendet werden. Dies ist in der Figur 13B dargestellt. Der Vorteil einer Einblendung des Warnzeichens 1395 im Bereich des schärfsten Sehens 1391 liegt darin, daß das Zeichen 1395 dort erscheint, wo das Auge hinschaut, und leitet dem Auge deshalb nicht dazu, von der vorliegenden Szene wegzuschauen. Aus diesem Grunde soll die Helligkeit eingeblendeter Zeichen SQ gewählt werden, daß die Zeichen transluzent erscheinen. Auf die Gefahr kann auch akustisch hingewiesen werden.

### Figur 14

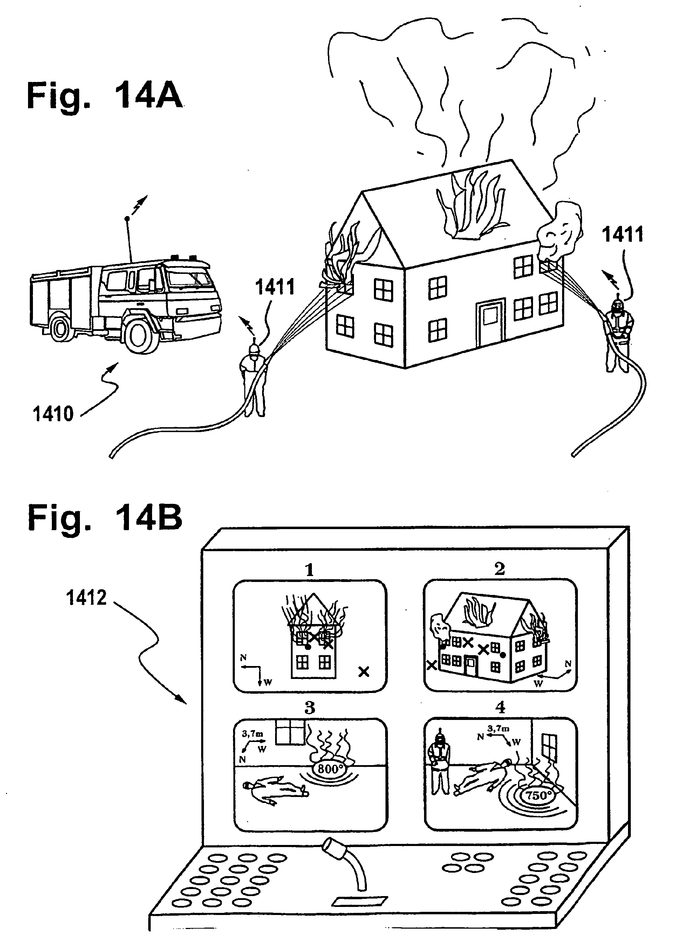

Die Figuren 14A und 14B zeigen ein Informationssystem gemäß einem zehnten Ausführungsbeispiel der vorliegenden Offenbarung, das die Möglichkeiten eines komplexen, vielseitigen Informationsystem verdeutlicht. Im konkret dargestellten Beispiel weist daß dargestellte Informationsystem eine mobile Feuerwehrleitzentrale 1410, die einen Kommandopult 1412 umfaßt, sowie mehrere Helmsysteme 1411 auf.

Jedes der Helmsysteme 1411 umfaßt eine wie zuvor beschriebene Signalerfassungsvorrichtung sowie eine Gesichtsfelderfassungsvorrichtung. Wahlweise kann jedes der Helmsysteme 1411 mit einer Rrojektionsvorrichtüng, Infrarotsensoren und/oder Lagesensoren ausgestattet werden. Sie können auch mit weiteren Sensoren ausgestattet werden, die beispielsweise eine Bestimmung der Luftqualität ermöglichen. Zu Kammunikationszwecken ist jeder der Helme 1411 beispielsweise mit einem Funkübertragungssystem ausgestattet, das mit oder Leitzentrale 1410 bzw. dem Kommandopult 1412 kommuniziert, und das durch sein Senden und Empfangen von Informationen sowohl Aufgaben einer Informationsvorrichtung als auch Aufgaben einer Ausgabevorrichtung übernimmt.

Bevorzugt werden die von den jeweiligen Helmen 1411 erfaßten Gesichtsfeldbilder, die auf der Basis der aus den Augen erfaßten Signale mit dem tatsächlich wahrgenommenen Gesichtsfeld des jeweiligen Feuerwehrmanns in Übereinstimmung gebracht werden können, an den Kommandopult 1412 übertragen und dort auf Monitore dargestellt. Zwecks einer Reduktion der zu übertragenden Datenmengen könnten Bediener des Kommandopults 1412 ebenfalls ein projizierendes Brillensystem tragen, damit lediglich die auf den Bereich der Fovea centralis des Bedieners fallenden Bilddaten in hoher Auflösung übertragen bzw. erfaßt werden müssen. In das Auge des Bedieners könnte ein korreliertes Gesichtsfeldbild eines einzelnen Feuerwehrmanns, oder ein Mosaik mehrerer Bilder hineinprojiziert werden. Dabei könnte der Bediener genau das sehen, was der Feuerwehrmann sieht, oder ein sich in Abhängigkeit von seinen eigenen Augenbewegungen veränderndes Bild aus dem Blickfeld des Feuerwehrmanns zur Verfügung gestellt bekommen.

Bei einer etwaigen Projektionen könnte dem Bediener und/oder dem Feuerwehrmann zusätzliches Information in das projizierte Bild hineingeflochten werden. Beispielsweise könnte durch die Lagesensoren bzw. Infrarotsensoren gewonnene Orientierungs- bzw. Temperaturinformationen in das Gesichtsfeld hineingeblendet werden. Die stetige Einblendung bestimmter Himmelsrichtungen, wie Nord und West, sowie von höhen Angaben wäre sowohl dem dem gesehenen Geschehen entfernten Bediener als auch dem durch Rauch und Qualm verschleierten Feuerwehrmann eine hilfreiche Bezugsangabe.

Durch eine entsprechende Aufbereitung der erfaßten Lageinformationen könnte aufgrund der inhärenten Vernetzung der Systemkomponenten jedem Feuerwehrmann die Position seiner Kollegen, beispielsweise mit einem kennzeichnendem "X", oder die Lage und Schwere der gesichteten oder auf sonstige Art erfaßten Feuerherde, beispielsweise mit einem der Feuerstärke entsprechend farbig gekennzeichneten Punkt , eingeblendet werden. Dies würde die Feuerbekämpfung erleichtern und die Wahrscheinlichkeit eines versehentlichen Verletztens eines hinter Rauch oder einer Wande unsichtbaren Kollegen verringern.

### Figur 15

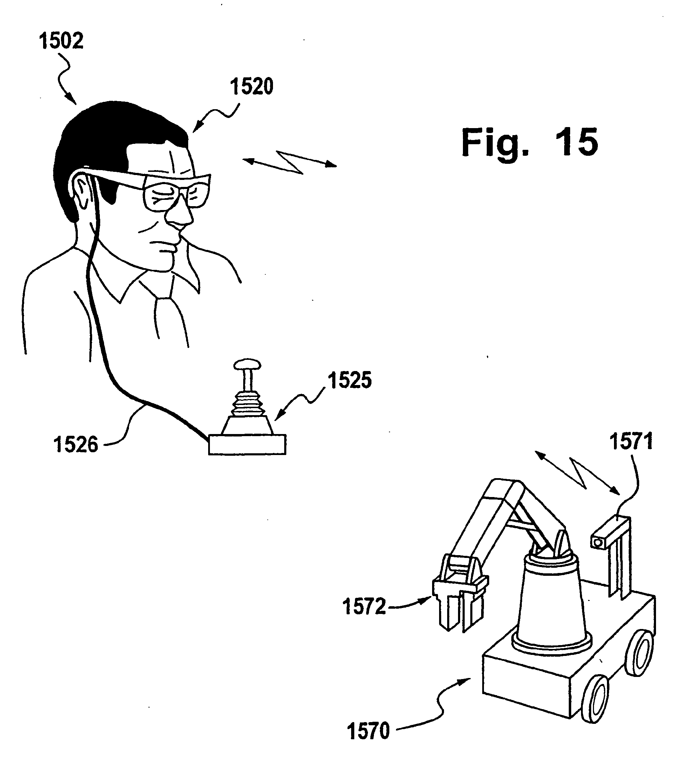

Die Figur 15 zeigt ein Informationssystem gemäß einem elften Ausführungsbeispiel der vorliegenden Offenbarung, bei dem das Informationsystem dem Bedienen eines externes System, beispielsweise eines zur Bewegung von gefährlichen Gegenständen ausgelegten, ferngesteuerten Roboters 1570, dient.

Gemäß der Abbildung umfaßt der auf Räder beweglicher Robot 1570 eine Kammeravorrichtung 1571 sowie einen Greifarm 1572. Der Roboter 1570 ist beispielsweise über eine Funkverbindung mit einem von einem Benutzer 1502 getragenen Brillensystem 1520 verbunden. Die über die Kameravorrichtung 1571 mono- oder stereoskopisch erfaßten Bilder ließen sich mit einer vom Brillensystem 1520 umfaßten Projektionsvorrichtung mono- bzw. stereoskopisch auf die Netzhaut des Benutzers 1502 projizieren. Bei einer stereoskopischen Projektion wäre ein räumliches Sehen gewährleistet.

Verfügt die Kameravorrichtung 1571 über eine makroskopische Objektiv, die ein breiteres "Gesichtsfeld" aufweist, als das Gesichtsfeld des Benutzers 1502, so kann das vom Benutzer 1502 gesehene Gesichtsfeld durch eine entsprechende Auswahl eines Bildausschnitts aus dem von der Kameravorrichtung 1571 gelieferten Bild, wie zuvor beschrieben, in Abhängigkeit von den erfaßten Augenbewegen des Benutzers 1502 mit dem fernen Bild in Korrelation gehalten werden. Ansonsten ließe sich die Kameravorrichtung 1571 in Korrelation mit den Augenbewegungen schwenken. Es können auch über Lagesensoren die Bewegungen des Kopfes des Benutzers 1502 derart erfaßt werden, daß die Kameravorrichtung 1571 in Korrelation mit den Kopfbewegungen mitschwenkt. Das Informationssystem gemäß der vorliegenden Offenbarung bietet somit ein bisher unerreichtes Maß an visueller Echtheit bei der Wahrnehmung einer entfernten Szene, was die Steuerung eines solchen externen Systems 1570 erheblich erleichtert.

Durch das Anbringen einer Mikrofon, insbesondere einer in Abhängigkeit der Kopfposition oder der Blickrichtung ausgerichteten Richtmikrofon, an das externe System in Verbindung mit einer Kopfhöreranordnung am Brillensystem läBt sich eine weitere sensorische Dimension realisieren.

Um eine weitere Bedienung des Roboters 1570 zu ermöglichen, ist ein manuell bedienbarer Steuerknüppel 1525 beispielsweise Über ein Kabel 1526 mit dem Brillensystem 1520 verbunden. Hierdurch ließe sich zum Beispiel den Greifarm 1572 oder die Fahrtrichtung des Roboters 1570 in mehrere Richtungen steuern.

### Figur 16

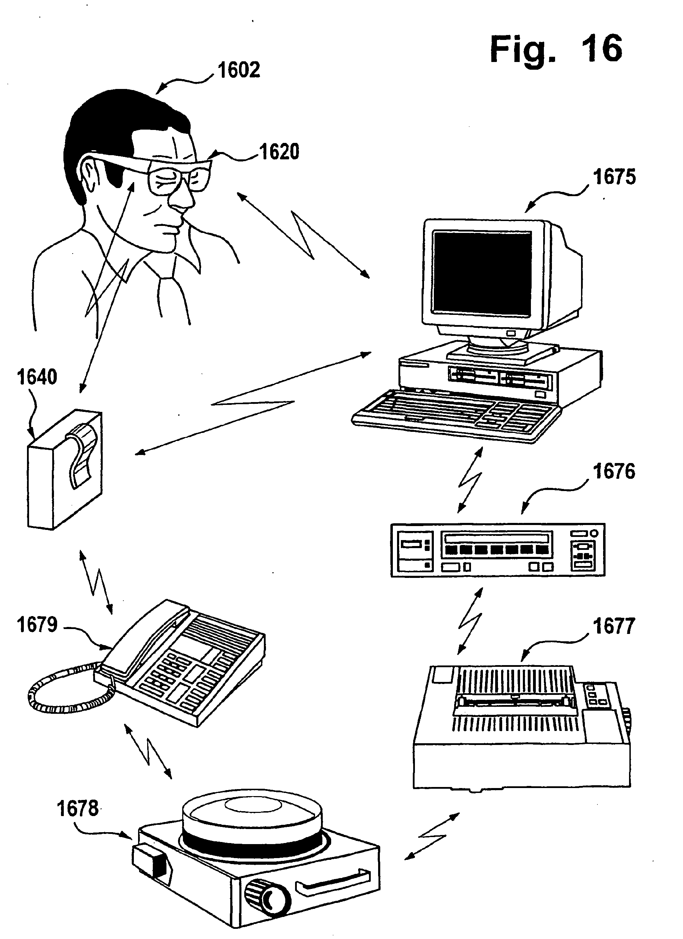

Die Figur 16 stellt ein Informationssystem gemäß einem zwölften Ausfuhrungsbeispiel der vorliegenden Offenbarung schematisch dar, bei dem ein Brillensystem 1620 als universelle Fernbedienung für ein oder mehrere Geräte fungiert, zum Beispiel ein Computer 1675, ein Videorekorder 1676, einen Drucker 1677, einen Diaprojektor 1678 und/oder ein Telefon 1679.

Im dargestellten System stellt das Brillensystem 1620 eine In zwei. Richtungen übertragende Schnittstelle zwischen einem Benutzer 1602 und dem zu bedienenden Gerät 1675-1679. Zuerst muß das Gerät 1675-1679 erkannt werden. Dies erfolgt im Sinne der der vorliegenden Offenbarung grundsätzlich über ein Anvisieren des zu bedienenden Geräts 1675-1679 mit der Fovea centralis. Die Identität des anvisierten Geräts 1675-1679 läßt sich entweder mit oder ohne die Mithilfe des Geräts 1675-1679 bestimmen. Im folgenden wird davon ausgegangen, daß sowohl das Gerät 1675-1679 als auch die Brille 1620 mit den für die beschriebenen Vorgänge notwendigen Signalempfangs- bzw. - sendevorrichtung ausgestattet werden.

Wird die Identität mit der Mithilfe des Geräts 1675-1679 bestimmt, so strahlt dieses Gerät 1675-1679 entweder in mehr oder minder regelmäßigen Intervallen ein Kennsignal aus, beispielsweise ein Infrarot- oder Ultraschallsignal, oder es wird von einer von der Brille 1620 ausgestrahlten Aufforderungssignal aufgefordet, ein Kennsignal auszustrahlen. Das Aufforderungssignal muß lokalisiert in Richtung Blickrichtung ausgestrahlt werden, um ein Ansprechen anderer Geräte zu vermeiden. Das vom Gerät 1675-1679 ausgestrahlte Kennsignal wird von der Brille erkannt, woraus auf die Identität des Gerätes geschlossen wird.

Wird die Identität ohne die Mithilfe des Geräts 1675-1679 bestimmt, so nimmt die Brille 1620 in Zusammenarbeit mit einer Datenbank oder sonstige Informationsquelle 1640, die Mustererkennungsdaten für die jeweilig ansprechbaren Geräte 1675-1679 enthält, eine Mustererkennung des anvisierten Bereichs des Gesichtsfelds vor.

Anhand der Identität des Geräts 1675-1679 wird ein der möglichen Funktionen des Geräts angepaßtes Menü in das Gesichtsfeld des Benutzers 1602 ggf. auf Tastendruck oder Augenzwinkern ortsfest eingeblendet. Ist die Funktionalität der Brille nicht ohne weiteres bekannt, so werden zuerst die entsprechenden Informationen aus einer Datenbank oder einer sonstigen Informationsquelle 1640, beispielsweise durch standardisierte Abfrage des Geräts selbst, in Kenntnis gebracht. Hier kann eine in das Abfragesignal eingebettete Identifizierung des Geräts dafür sorgen, daß lediglich das gewünscht Gerät auf die Abfrage antwortet. Dadurch, daß das Menü ortsfest in das Gesichtsfeld eingeblendet wird, kann der Benutzer 1602 das ggf. hierarchische Menü durch geringfügige Augenbewegungen wie ein Computermenü bedienen.

Nachdem die gewünscht Funktion ausgewählt worden ist, wird ein der Funktion entsprechendes Signal von der Brille 1620 an das Gerät 1675-1679 gesandt. Hier kann eine in das Signal eingebettete Identifizierung des Geräts dafür sorgen, daß lediglich das gewünscht Gerät auf das Signal reagiert.

Auf diese Art und Weise könnte mit geringer Hardwareaufwand eine schnelle und einfache Bedienung vieler Geräte erzielt werden.

### Figur 17

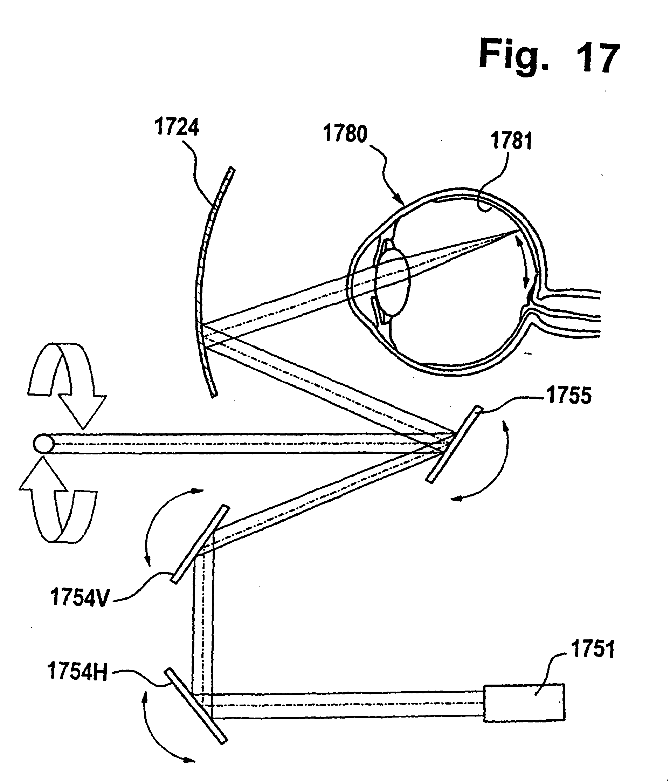

Die Figuren 17 zeigt ein optisches System gemäß einem dreizehnten Ausführungsbeispiel, bei dem ein Kippspiegel 1755 ein Umschalten zwischen einer Aufnahme aus dem Gesichtsfeld und einer Aufnahme aus dem Auge 1780 oder einer Projektion auf die Netzhaut 1781 ermöglicht.

Der Vorteil dieses optischen Systems liegt darin, daß die gleichen Taumelspiegel 1754H und 1754V für eine Aufnahme aus dem Gesichtsfeld und für eine Projektion auf die Netzhaut 1781 verwendet werden kann, und daß der Strahlengang für eine Aufnahme aus dem Gesichtsfeld und der Strahlengang für eine Aufnahme aus dem Auge 1780 bzw. eine Projektion auf die Netzhaut 1781 dementsprechend zum Großteil identisch sind. So wird schon durch das optische System eine hohe Korrelation zwischen dem aus dem Gesichtsfeld erfaßte Licht und den aus dem Auge erfaßten Signale bzw. eine hohe Korrelation zwischen dem aus dem Gesichtsfeld erfaßte Licht und dem auf die Netzhaut projizierte Bild erzielt. Das heißt, es werden keine zusätzliche Korrelationsfehler dadurch versucht, daß die besprochenen Strahlengänge über verschiedene Taumelspiegel verlaufen, die unterschiedliche Rotationscharakteristika aufweisen könnten. Für Lichterfassung aus dem Gesichtsfeld und Lichterfassung aus dem Auge kann sogar die gleiche Lichterfassungvorrichtung 1751 verwendet werden. Lediglich durch die Reflektion am Brillenglas 1724 und das optische System des Auges 1780 kann die Korrelation negativ beeinflußt werden.

### Nicht-dargestellte Ausführungsbeispiele

Ergänzend zu den in den Figuren dargestellten Ausführungsbeispielen werden nachstehend weitere möglichen Ausführungsformen des Informationssystem gemäß der vorliegenden Offenbarung zwecks einem besseren Verständnis der Erfindung beschrieben

### TV / Zeitung

Bisherige elektronische Bücher bzw. Zeitungen haben den Nachteil, zu schwer und/oder zu unhandlich zu sein, und können außerdem nur eine begrentze Informationsmenge pro Seite darstellen. Auch tragbare Video- und Fernsehgeräte sind schwer und/oder unhandlich. Wird das Informationssystem gemäß der vorliegenden Offenbarung derart ausgebildet, daß das Zuverfügungstellen von Informationen eine Projektion von Bildinformationen in das Auge umfaßt, so lassen sich verschiedene visuell bezogene Medien, beispielsweise elektronische Bücher oder Zeitungen, Fernsehen oder Videospiele, durch das Informationssystem verwirklichen. Dabei wird das Informationssystem gemäß der vorliegenden Offenbarung zum Beispiel, wie oben beschreiben, in Form einer tragbaren Brille realiziert, die über eine Kabel-, Infrarot- oder Funkverbindung beispielsweise an ein Informationsnetz, eine tragbare Speichervorrichtung, zum Beispiel ein CD-ROM- oder DVD-Lesegerät, oder eine sonstige Informationsquelle angeschlossen werden kann.

Ein Vorteil einer derartigen Ausbildung des Informationssystems gemäß der vorliegenden Offenbarung liegt darin, daß seine Erfassung von Signalen aus dem Auge in Zusammenhang mit seiner Gesichtsfelderfassung eine Projektion ermöglicht, bei dem der projizierte Text bzw. die projizierte Bilder im Raum fixiert zu sein scheint. Zu diesem Zweck umfaßt die Informationsvorrichtung eine Auswertevorrichtung, die die Korrelation der Sehachse zum Blickfeld ermittelt, und die die Projektion entsprechend steuert, so daß die auf das Auge projizierten Informationen vis-à-vis dem Blickfeld trotz Bewegungen des Auges unbeweglich zu sein scheinen. Die Ermittlung der Korrelation der Sehachse zur Umgebung kann auch durch in der Brille angebrachte Lagesensoren unterstützt werden.

Der virtuelle 0rt der Fixierung kann beispielsweise über eine Fixierung mit den Augen in Zusammenhang mit einem Augenzwinkern oder Tastendruck oder auch automatisch, zum Beispiel mittels einer bildverarbeitenden Auswertung des Blickfelds, die ein möglichst inhaltsarmes Gebiet des Blickfelds ermittelt, festgelegt werden. Die störende Wirkung des durch die Projektion der Informationen nicht notwendigerweise abgedeckten, natürlichen Gesichtsfeldes ließe sich durch ein farbkomplementäres "Auswischen" verringern, bei dem komplementärfarbige Bildpunkte anhand des aus dem Gesichtsfeld erfaßten Lichts ermittelt werden, deren korrelierte Projektion auf die jeweilig zugeordnete Gebiete der Netzhaut den natürlichen Hintergrund durch Farbaddition als weiß erscheinen läßt. Ist ein schwarzer Hintergrund erwünscht, so muß, wie zuvor beschrieben, die empfundene Gesamthelligkeit der Projektion die empfundene Gesamthelligkeit des natürlichen Gesichtsfeldes um ca. 10% bis 20% überschreiten, damit auch die hellsten Punkte des natürlichen Gesichtsfeldes als schwarz empfunden werden.

Zu Bedienungszwecken könnten Bildinformationen, die virtuelle Bedienungsknöpfe darstellen, derart in das Auge hineinprojiziert werden, daß sie in der Nähe des Textes bzw. Bildes im Gesichtsfeld ebenso fixiert erscheinen. Somit ließe sich das virtuelle Informationsmedium mittels Anvisierten des entsprechenden Bedienungsknopfes mit der Fovea centralis plus Tastendruck oder Augenzwinkern fernbedienen, d.h. Umblattern, Vorspulen, Zurückspulen, o.ä. Ähnlich könnte ein Zugriff auf Lexika, Datenbanken, u.s.w. durch das Anvisieren von dargestellten Wörtern oder Bildteile ermöglicht werden. Anstatt Bedienungsknöpfe ließe sich das Informationssystem beispielsweise auch über eine Menüführung bedienen, bei der Bedienmenüs bei der Betrachtung bestimmter Bildbereich "aufspringen", um ein augengesteuertes Auswählen aus dem ggf. hierarchisch aufgebauten Menü zu ermöglichten.

Ein weiterer Vorteil einer derartigen Ausbildung des Informationssystems liegt darin, daß die für eine ausreichende momentane Darstellung notwendige Datenmenge bei weitem geringer ist, als die Datenmenge, die für hochauflösende Darstellung des gesamten Gesichtsfeldes notwendig wäre. Dies liegt der Tatsache zugrunde, daß das Informationssystem den Bereich des schärfsten Sehens kennt. Somit müssen nur diejenigen Teile der Projektion mit hoher Auflösung erfolgen, die den Bereich der Fovea centralis betreffen. Auf sonstige Gebiete der Netzhaut genügt eine Projektion mit geringer Bildpunktdichte. Dementsprechend reduziert sich die für eine momentane Darstellung notwendige Datenmenge, was deutliche Systemvorteile mit sich bringt. Insbsondere läßt sich die empfundene Größe des projizierten Bildes beliebig wählen, ohne daß unbearbeitbar groß Datenmengen zur Präsentation des momentanen Bildes die Folge sind.

Ist das projizierte Bild größer als das Gesichtsfeld, dann bestimmt die momentane Sehachse den Bildausschnitt. Die Projektion erfolgt derart, daß der aktuelle Bildausschnitt den gesamten aktiven Bereich der Netzhaut füllt. Durch Augenbewegung können weitere Ausschnitte des Bildes in das Gesichtsfeld hineingebracht werden. Ist das projizierte Bild kleiner als das Gesichtsfeld, so muß lediglich auf einen beschränkten Teil der Netzhaut projiziert werden. Wird der natürliche Gesichtfeldhintergrund nicht ausgeblendet, so ändert sich dieser bei Augenbewegungen. Insbesondere bei fernseh- oder kinoartigen Informationsdarstellungen ist eine das Gesichtsfeld genau füllende Projektion bevorzugt.

Werden Signale aus beiden Augen eines Benutzers erfaßt, so kann die Projektion stereoskopisch erfolgen, wobei jedem Auge ein derart geringfügig unterschiedliches Bild zugespeist wird, daß das Gehirn ein dreidimensionales Gesamtbild wahrzunehmen glaubt. Somit ließe sich eine optimale System-Mensch-Schnittstelle beispielsweise für 3D-Fernsehen, 3D-Videospiele, 3D-CAD-Anwendungen oder sonstige, insbesondere interaktive, 3D-Anwendungen verwirklichen. Bevorzugt umfaßt das Informationssystem weitere Bedienelemente, zum Beispiel ein Steuerknuppel, Pedal oder Lenkrad, die eine Navigation bzw. Perspektivwechsel innerhalb des dargestellten virtuellen Bildes oder eine sonstige Beeinflussung der Informationsdarbietung oder eines mit der Informationssystem verbunden Systems ermöglicht. Wie zuvor beschrieben, kann auch das Auge selbst als Bedienelement fungieren.

Unter entsprechender Anwendung der vorstehend für die Positionierung einer elektronischen Zeitung an einem virtuellen Ort erforderlichen Maßnahmen ist es gleichermaßen möglich, dem Träger des Informationssystems andere Orientierungshilfen auf die Netzhaut zu spielen, wie z.B. einen künstlichen Horizont.

### Ophthamologische Anwendungen / Sehhilfen

Aufgrund ihrer Erfassung aus dem Auge zurückreflektierter Signale eignet sich das Informationssystem gemäß der vorliegenden Offenbarung hervorragend zur Ausgestaltung als ophthamologisches System. Zum Beispiel läßt sich das Informationssystem als Positioniersystem für die ophthamologische Chirugie, insbesondere für die ophthamologische Laserchirugie, realisieren. Auch als ophthamologisches Diagnosesystem, Sehhilfesystem und/oder Sehfehlerkorrektursystem findet das Informationssystem beispielsweise Anwendung.

Die meisten Strukturen oder Organe des Auges sind im Vergleich zu manuellen Bewegungen sehr kein. Erkrankungen und Beschädigungen dieser Strukturen bzw. Organe betreffen häufig nur einen kleinen, mikroskopischen Bereich. Im Gegensatz zu vielen anderen Körperpartien lassen sich die Augen jedoch nicht fixieren, was die Behandlung evtl. Erkrankungen oder Verletzungen des Auges besondere erschweren.

Aufgrund der Fähigkeit des Informationssystems, Bewegungen des Auges genau verfolgen und Informationen bezüglich der augenblicklichen Stellung des Auge auch anderen Systemen zur Verfügung stellen zu können, lassen sich diese Schwierigkeiten durch ein therapeutisches System auf der Basis des Informationssystems überwinden. Zum Beispiel kann das therapierende System derart mit dem Informationssystem zwecks Informationsaustausch verbunden sein, daß Informationen bezüglich der augenblicklichen Stellung des Auge dem therapierenden System zur Verfügung gestellt werden, so daß eine punktgenaue automatisierte Therapie des Auges auch bei bewegten Augen erfolgen kann.

Gemäß einem anderen Ausführungsbeispiel wird ein therapierender Laserstrahl über das optische System des Informationssystems gelenkt. Eine Laserbehandlung des Auges, insbesondere der Netzhaut, kann somit auf gleiche Art wie eine wie zuvor beschriebene Projektion erfolgen. Beispielsweise können krankhafte Adern der Aderhaut dadurch verödet werden, daß ein photoempfindliches Mittel eingespritzt oder eingenommen wird, und daß krankhafte Stellen der Aderhaut über mehreren Zehn Sekunden punktgenau bestrahlt werden. Eine derartige Therapie läßt sich mit Hilfe das Informationssystems präzis ausführen.

Um als Sehhilfe- und/oder Sehfehlerkorrektursystem Anwendung zu finden, umfaßt die Ausgabevorrichtung des Informationssystems eine Projektionvorrichtung, die sichtverbessernde Bildinformationen auf die Netzhaut projiziert. Zudem wird die Informationsvorrichtung eine Auswertevorrichtung umfassen, die die sichtverbessernde Bildinformationen anhand des aus dem Gesichtsfeld erfaßten Lichts ermittelt. Die sichtverbessernden Bildinformationen werden bevorzugt derart in Korrelation mit den aus dem Auge erfaßten Signalen auf die Retina projiziert, daß das natürlich wahrgenommene Gesichtsfeld und die projizierten Bildinformationen als einheitliches Bild wahrgenommen werden. Im Extremfall werden die sichtverbessernden Bildinformationen derart auf die Retina projiziert, daß das ansonsten natürlich wahrgenommene Gesichtsfeld vom Auge gar nicht wahrgenommen wird. Wie zuvor beschrieben, kann der Grad der Wahrnehmung eines so projizierten Bildes im Verhältnis zum natürlich wahrgenommenen Bild durch die Helligkeit der projizierten Bildpunkte gesteuert werden.

Durch ein derartiges Informationssystem läßt sich beispielsweise eine Sehfehlerkorrektur für Kurz- oder Weitsichtige sowie bei Farbsehschwäche durchführen. Bei der Korrektur einer Kurz- bzw. Weitsichtigkeit kann das Informationssystem auf eine (quasi- )festen Korrektur eingestellt werden, eine veränderbare Korrektur ermöglichen, oder sich dynamisch auf den Sehfehler automatisch einstellen. Die Korrektur erfolgt über ein ggf. einstellbares optisches Fokussiersystem innerhalb der Projektionsvorrichtung oder durch bildverarbeitende Maßnahmen. Letzteres läßt sich mit geringem Systemaufwand realisieren.

Implementierungen mit (quasi-)fester oder veränderbarer Korrektur sind durch ihre inhärente Ähnlichkeit zu ähnlichen optischen Systemen für den Fachmann ohne weitere Erklärung verständlich. Eine Realisierung mit einer dynamischen, automatischen Korrektur des natürlichen Abbildungsfehlers umfaßt neben der oben beschriebenen Korrelation eine weitere Abhängigkeit zu den vom Auge erfaßten Signalen. Insbesondere wird dabei ein Netzhautreflexbild erfaßt, das durch Vergleich mit dem aus dem Gesichtsfeld erfaßten Licht und/oder durch eine bildverarbeitende Auswertung Auskunft über die Schärfe des auf der Netzhaut abgebildete Bild liefert. Entsprechend wird das aus dem Gesichtsfeld erfaßte Licht in sichtverbessernde Bildinformationen aufbearbeitet und auf die Retina projiziert. Durch Ausgabe des so ermittelten Korrekturwertes kann das Informationssystem als Diagnosesystem fungieren.

Durch seine Erfassung aus dem Auge zurückreflektierter Signale und aus dem Gesichtsfeld stammenden Lichtes ist das Informationssystem mittels einer entsprechend programmierten Auswertevorrichtung in der Lage, Auskunft über viele ophthamologisch relevanten Eigenschaften des Auge zu geben. Zum Beispiel lassen sich Schielwinkel, Primär-Positionen (PP), Gesichtsfeldbestimmungen auch mit Farben, Schwellwerttests, standardisierte Testverfahren für Glaukomadiagnose, Prüfungen von Netzhautfunktionen (beispielsweise ERG und VEP) auch an ausgewälten Orten und Prüfungen der rezeptiven Felder durchführen bzw. bestimmen. Die hierzu zu erfassende Signale aus dem Auge, die hierzu notwendigen Gesichtsfeldreize und die hierzu notwendigen Bearbeitungsalgorithmen wählt der Fachmann auf der Basis seiner Fachkenntnis und unter Berücksichtigung der vorstehend beschriebenen Ausführungsbeispiele entsprechend aus.

Während beispielsweise die Sehschärfe sich durch eine Auswertung aus dem Auge zurückreflektierter Signale feststellen und anschließend korrigieren läßt, setzt die Körrektur manch anderer Sehfehler eine systemunabhängige Feststellung des Fehlers, zum Beispiel durch einen Augenarzt, voraus. Eine passende Einstellung der durch das Informationssystem vorgenommenen Korrektur kann rekursiv oder einfach durchgeführt werden.

Bei einem rekursiven Einstellvorgang wird eine Korrektur gemäß vorheriger Einstellung vom Informationssystem vorgenommen währende das Sehvermögen der fehlsichtigen Person getestet wird. Anhand der Ergebnisse der Tests wird eine neue Einstellung des Informationssystems gewählt. Dieser Vorgang wird wiederholt durchgeführt, bis der Sehfehler ausreichend kompensiert worden ist. In diesem Sinne fungiert das Informationssystem gleichwohl als Diagnosesystem; denn anhand der bestkorrigierenden Endeinstellung kann der Sehfehler bestimmt werden.

Bei einem einfachen Einstellvorgang wird das Sehvermögen der fehlsichtigen Person ohne jeglicher Kompensation getestet. Anhand der Ergebnisse der Tests wird eine passende Einstellung des Informationssystems gewählt, das im späteren Einsatz das aus dem Gesichtsfeld erfaßte Licht dann gemäß dieser Einstellung in sichtverbessernde Bildinformationen auf bearbeitet und auf die Retina projiziert. Bei der Aufbereitung werden, der Einstellung, d.h. dem ursprünglichen Sehfehler, entsprechend, beispielsweise bestimmte Spektralkomponenten oder bestimmte Bereiche des Gesichtsfeldes hervorgehoben oder durch sonstige bildverarbeitenden Maßnahmen verändert.

für Nachtblinde kann zum Beispiel eine Sehhilfe durch das Informationssystem dadurch verwirklicht werden, daß das aus dem Gesichtsfeld, beispielsweise durch stark lichtempfindliche Photodetektoren, erfaßte Licht stark verstärkt auf die Retina projiziert wird. Dabei können die Zapfen derart angeregt werden, daß ein überwiegend farbiges, photopisches Sehen statt ein skotopisches Sehen stattfindet. Es wird auch die maximal erlaubte Helligkeit der einzelnen projizierten Bildpunkte auf einen vorgegebenen Schwellwert beschränkt, um ein Blenden durch hell leuchtende Gegenstände wie Straßenlaternen und entgegenkommenden Autos zu vermeiden. Ein solches System eignet sich also auch als Anti-Blend-System. Denn wird die Helligkeit des gesamten Gesichtsfelds gehoben, während die "übermäßige" Helligkeit einzelner Punkte unverändert bleibt, so werden die "übermäßig" helle Punkte nicht mehr als "übermäßig" hell empfunden. Umfaßt die Informationsvorrichtung auch einen Infrarotsensor, der Infrarotlicht aus dem Gesichtsfeld erfaßt, so lassen sich zusätzliche einfarbige Bildinformationen bezüglich des Gesichtsfelds bei Nacht oder Nebel gewinnen, die in den sichtbaren Spektralbereich transformiert werden können, um die schon mittels der Gesichtfelderfassungsvorrichtung und der Auswertevorrichtung gewonnen Bildinformationen aufzuwerten.

Auch im Allgemeinen kann das Informationssystem dazu geeignet sein, die Sehfähigkeit zu verbessern. Beispielsweise bei starken oder schwachen Kontrasten oder bei geringer Helligkeit im Gesichtsfeld können in ihrer Helligkeit angepaßte Bildinformationen in das Auge projiziert werden, um eine verbesserte Sehfähigkeit zu ermöglichen.

### Helme

Die Integration des Informationssystems in einem Feuerwehrmannheim wurde oben erläutert. Ähnliche Ausgestaltungen, beispielsweise als Soldaten-, Fahrer-, Kranfahrer-, Sportler- oder Pilotenhelm oder -brille sind ebenfalls denkbar.

Ein Soldatenhelm bzw. -brille auf der Basis des Informationssystems könnte dem Soldaten zum Beispiel bei der Orientierung und/oder bei der Zielsuche behilflich sein. In einem solchen Fall umfaßt die Informationsvorrichtung des Informationssystems bevorzugt Sensoren und/oder Funkempfänger,eine übersinnliche Wahrnehmung der Umgebung und/oder das Empfangen von Informationen von einer Kommandozentrale ermöglichten. Die Ausgabevorrichtung wird Informationen bevorzugt visuelle, hörbar oder taktil, zum Beispiel in Form kurzer elektrischer Reizströme an der Haut, zur Verfügung stellen. Letzteres könnte dazu verwendet werden, einen Soldaten unmittelbar über die Richtung eines von hinten zubewegenden Fremdobjekt zu informieren.

Als Nachtsichtgerät würde das Informationssystem neben der Erfassung von sichtbarem Licht aus dem Gesichtsfeld auch Infrarotlicht aus dem Gesichtsfeld erfassen. Wie zuvor beschrieben, können Bildinformationen aus solch erfaßtem Infrarotlicht gewonnen und bei der Aufwertung von in das Auge zu projizierenden Bildinformationen eingesetzt werden.

Weist die Informationsvorrichtung beispielsweise einen GSP-Empfänger auf, so könnte der Helm Positionsinformationen oder Orientierungshilfen auf die Netzhaut projizieren. Bevorzugt erfolgt die Projektion solcher Informationen ins Auge ähnlich der Projektion einer elektronischen Zeitung. Das heißt, es wird eine Ablenkung des Soldaten dadurch vermieden, daß das Bild der Informationen im Raum oder vis-ä-vis einer neutralen Stellung des Auges fixiert zu sein scheint. Auch eine Anpassung der Bildinformationen an den dahinter wahrgenommenen Hintergrund zwecks einer möglichst guten Lesbarkeit findet durch eine zur Informationsvorrichtung gehörende Auswertevorrichtung statt.

Auch wenn eine Funk- oder sonstige Datenübertragung vom Soldaten aus an eine Kommandozentrale aus strategischen Tarnungsgründen generell zu vermeiden gilt, könnte in bestimmten Fällen auch eine Übertragung von mit den Augenbewegungen des Soldaten korrelierte Gesichtfelddaten an eine Kommandozentrale sinnvoll sein.

In einer für Soldaten besonders interessanten Ausführungsform umfaßt die Informationsvorrichtung eine oder mehrere Kameras, die Bilder von außerhalb des Gesichtsfeldes erfassen. Die so gewonnenen Bildinformationen werden dann über eine Projektionsvorrichtung auf die Retina projiziert. Das auf das Gesichtsfeldbild projizierte Zusatzbild könnte zum Beispiel als Bild im Bild als kleines Bild in die Ecke des natürlichen oder projizierten Gesichtsfeldbildes projiziert werden oder als Längstreifen am unteren Rand erscheinen. Dabei dient die Erfassung von Signalen aus dem Auge zusammen mit der Gesichtsfelderfassung dazu, die projizierten Bilder in Korrelation mit den Bewegungen des Auges zu halten.

Beim Kranfahrer wäre es ebenfalls hilfreich, Zusatzbilder aus anderen Perspektiven in das Gesichtsfeld hineinzuprojizieren. Gleichfalls könnte das Informationssystem Zusatzsensoren umfassen, mit deren Hilfe Entfernungs- oder Gewichtsinformationen ermittelt werden, um in das Gesichtsfeld hineinprojiziert zu werden. Solche Informationen können beispielsweise auch beim Anblick der Last in Kombination mit einem Tastenklick hörbar oder visuell zur Verfügung gestellt werden. Dabei dient das aus dem Gesichtsfeld ermittelte Licht als Grundlage der Bilderkennung während die Signale aus dem Auge wie zuvor beschrieben eine Korrelation des erfaßten Gesichtsfelds zur Sehachse ermöglichen.

Einem Piloten könnte das Informationssystem viele verschiedenen Informationen zur Verfügung stellen. Durch eine Anbindung an das Informationssystem des Flugzeugs könnten zum Beispiel relevante Daten wie Flughöhe, Geschwindigkeit oder Flugrichtung oder auch ein künstlicher Horizont in das Gesichtsfeld des Piloten wie beschrieben eingeblendet werden. Beim Anflug könnten zudem Landehilfeinformationen eingeblendet werden, die einen virtuellen Landekörridor darstellen, oder Höhen- oder Richtungskorrekturwerte angeben. Bei militärischer Abwendung können dem Pilot Freund/Feind- und Zielhilfeinformationen zur Verfugung gestellt werden. Hier spielt die Blickrichtung des Piloten sowohl bei der räumlichen Einblendung der Informationen als auch bei der Informationsauswahl eine Rolle. Der Pilot möchte, daß ein Flugkörper, den er mit den Augen anvisiert, identifiziert wird. Falls die Idenifizierung visuell erfolgt, möchte er, daß die Einblendung keine relevanten Bereiche seines Gesichtsfeldes überdecken. Dabei sind die gegenläufige Anforderungen zu berücksichtigen, daß die relevanten Bereiche des Gesichtsfeldes typischerweise auf der Fovea centralis abgebildet werden, aber auch, daß nur diejenigen Bilder, die auf die Fovea centralis projiziert werden, scharf abgebildet werden. Es muß also eine intelligente Einblendung erfolgen, bei der die relevanten Bereiche des Gesichtsfelds zum Beispiel über eine Bilderkennung und nicht lediglich über die Ausrichtung der Fovea centralis erkennt werden. In diesem Zusammenhang kann das Informationssystem gemäß der vorliegenden Offenbarung auch als Untersystem zum Informationssystem des Flugzeugs fungieren und diesem Informationenen zur Verfügung stellen. So könnten beispielsweise Informationen darüber, wo der Pilot hinschaut, vom Informationssystem an das Flugzeuginfomationssystem geliefert werden und dort zur Zielerfassung beitragen. Im Ernstfall könnte das Informationsystem feindliche Radarstellung über Sensoren orten und ihre Position mit dem dazugehörigen Gelände dreidimensional darstellen.

Sportlern könnten durch das Informationssystem wie in den vorhergehenden Beispielen verschiedene Informationen zur Verfügung gestellt werden. Mittels einer Projektion von Informationen in das Auge könnten beispielsweise Orientierungshilfen, Geschwindigkeitsinformation und/oder aufgewertete Gesichtfeldinformationen, die eine bessere Sicht bei Dämmerung, Nacht, Regengischt oder Nebel ermöglichen, zur Verfügung gestellt werden. Insbesondere bei gehaltsarmen Informationen eignet sich ein nicht visuelles Zurverfügungstellen der Informationen. Ähnlich den vorhergehenden Beispielen kann ein von einem Sportler getragenes Informationssystem als Untersystem eines Sportgerätes oder eines Fahrzeuges fungieren.

### Reine Informationssysteme

Eine übersinnliche Wahrnehmung läßt sich durch Ausführungsformen des Informationsystems gemäß der vorliegenden Offenbarung erzielen, bei den die Informationsvorrichtung einen oder mehrere Sensoren beispielsweise Magnetfelddetektoren, Drucksensoren, Thermometer, Spektralsensoren, optische oder akustische Interferenzmeßgeräte umfaßt. Insbesondere durch eine Überlagerung einer in das Auge projizierten, bildlichen Darstellung der aus den Sensoren gewonnenen Informationen auf das natürliche Gesichtsfeld entspricht die Darstellung den Bedürfnissen eines sehenden Menschen. Dabei kann das Informationssystem als Bestandteil, insbesondere als Präsentationseinrichtung, einer komplexen Meßeinrichtung auftreten.

Als Beispiel eines solchen Systems gilt ein mit empfindlichen Magnetsensoren ausgestattetes Brillensystem, das in der Lage ist, stromführende oder metallische Gegenstände in Relation zur Brille zu orten. Werden solche georteten Gegenstände mittels einer wie zuvor beschriebenen Projektion ortsgetreu im natürlichen Gesichtsfeld farbig gekennzeichnet, so ließen sich beispielsweise unter Putz verlaufende Wasser- oder Stromleitungen sehr leicht auffinden. Ein ein solches Brillensystem tragender Monteur würde den Verlauf der Leitungen sozusagen "an der Wand gepinselt" sehen.

Wird ein zwei- oder dreidimensionales Array oder sonstige ein- oder mehrdimensionale Verteilung der Sensoren gewährt, so können auch beispielsweise sehr komplexe Vektorfelder oder Gradientenverläufe einem Betrachter bildlich über den dazugehörigen Gegenstand oder Anordnung sichtbar gemacht werden. Zum Beispiel könnte eine Anordnung von Drucksensoren um ein Testobjekt in einem Windtunnel herum Druckinformationen liefern, die durch das Informationsystem wie zuvor beschrieben derart aufbereitet und in die Augen eines Betrachters, der das Testobjekt durch ein Fenster beobachtet, projiziert, daß er die durch das Testobjekt entstehenden Druckgradienten anhand entsprechender farbiger Kennzeichnung der Druckwerte dort sieht, wo sie vorhanden sind. Einem schweißen könnten mittels einer Infrarotkamera gewonnenen Temperaturinformationen derart in sein Gesichtsfeld dargestellt werden, daß die örtliche Oberflächentemperatur entlang den bearbeiteten Gegenstände erkenntlich ist.

Ähnlich können Spektralsensoren dazu verwendet werden, einem Benutzer Auskunft über genaue Farbwerte oder Materialzusammensetzungen zu geben. Hier bietet es sich auch an, die ermittelten Informationen, in Abhängigkeit davon, wo der Benutzer genau hinschaut, hörbar zu präsentieren. In Zusammenarbeit mit einer Datenbank und einer Mustererkennung könnte ein solches System zum Beispiel dazu verwendet werden, Pilze oder Pflanzen zumindest annähernd zu identifizieren, indem der Benutzer bestimmte Teile des Pilzes bzw. der Pflanze auf Systemaufforderung anschaut bzw. den Sensoren zuwendet.

## Patentansprüche

1. System zum Zurverfügungstellen von Informationen, mit
einer In einem ersten Koordinatensystem sich befindenden, ersten optischen Signalerfassungseinrichtung (551) zur Erfassung von Licht, das von einem Auge zurrückreflektiert oder abgestrahlt worden ist;
einer in dem ersten Koordinatensystem sich befindenden, zweiten optischen Signalerfassungseinrichtung (1061L, 1061R) zur Erfassung von Licht aus der natürlichen Umgebung des Auges, ohne Licht zu erfassen, das vom Auge reflektiert worden ist;
einer Auswertevorrichtung, die die Blickrichtung des Auges bezüglich des ersten Koordinatensystem aus dem von der ersten optischen Signalerfassungseinrichtung erfaßten Licht bestimmt, die Lage und Orientierung des ersten Koordinatensystems aus dem von der zweiten optischen Signalerfassungseinrichtung erfaßten Licht ermittelt, und somit eine Korrelation zwischen dem Auge und der Umgebung herstellt;
einer Ausgabevorrichtung, die Information anhand der Korrelation zwischen dem Auge und der Umgebung zur Verfügung stellt, wobei
die Ausgabevorrichtung eine Projektionsvorrichtung (553) umfaßt, die die Information in Form von sichtbaren Bildern in das Auge projiziert, und
das von der zweiten optischen Signalerfassungsvorrichtung erfaßte Licht dazu verwendet wird, die sichtbaren Bilder derart in das Auge zu projizieren, daß die sichtbaren Bilder bei einer Bewegung des Auges festzustehen scheinen.

2. System nach Anspruch 1, mit
einer Informationsvorrichtung, die Bildinformationen aus dem von der zweiten optischen Signalerfassungseinrichtung erfaßten Licht gewinnt, wobei
die Ausgabevorrichtung die Informationen auch in Abhängigkeit von der gewonnenen Bildinformationen zur Verfügung stellt.

3. System nach Anspruch 2, wobei die Informationsvorrichtung die Bildinformationen aus dem von der zweiten optischen Signalerfassungseinrichtung erfaßten Licht anhand von Ortungsinformationen gewinnt.

4. System nach einem der vorhergehenden Ansprüche, wobei die Auswertevorrichtung die Orientierung des Auges bezüglich der natürlichen Umgebung des Auges ermittelt.

5. System nach einem der vorhergehenden Ansprüche, wobei die Projektionsvorrichtung die sichtbaren Bilder derart In das Auge projiziert, daß die natürliche Umgebung des Auges und die sichtbaren Bilder als einheitliches Bild wahrgenommen werden.

6. System nach einem der vorhergehenden Ansprüche, mit einem Kommandopult (1412), wobei die erste und zweite Signalerfassungseinrlchtungen Teil eines Helmsystems sind.

7. System nach einem der vorhergehenden Ansprüche, mit einem Lagesensor, insbesondere einem GPS-Empfänger und/oder einer Beschleunigungsmeßvorrichtung.

8. System nach Anspruch 7, mit einer Datenbank (1640) oder Datenbankanbindung, die Orientierungsdaten liefert.

9. System nach einem der vorhergehenden Ansprüche, wobei die erste und zweite optische Signalerfassungseinrichtung Teil einer Brille sind, die das erste Koordinatensystem darstellt.

10. System nach einem der vorhergehenden Ansprüche, wobei die erste Signalerfassungseinrichtung regelmäßig eine bearbeitungsarme Signalerfassung und intermittierend eine bearbeitungsintensive Signalerfassung durchführt.

## Claims

1. A system for providing information, comprising:
a first optical signal capturing apparatus (551) located in a first coordinate system for capturing light that has been reflected or emitted from an eye;
a second optical signal capturing apparatus (1061L, 1061R) located in said first coordinate system for capturing light from the natural environment of the eye without capturing light that has been reflected from said eye;
an evaluation apparatus that determines the viewing direction of the eye relative to said first coordinate system from said light captured by said first optical signal capturing apparatus, ascertains the location and orientation of said first coordinate system from said light captured by said second optical signal capturing apparatus, and thus establishes a correlation between said eye and said environment;
an output apparatus that provides information based on said correlation between said eye and said environment, wherein
said output apparatus comprises a projection apparatus (553) that projects said information into said eye in the form of visible images, and
said light captured by said second optical signal capturing apparatus is used to project said visible images into said eye such that said visible images appear to be fixed during movement of said eye.

2. The system of claim 1, comprising:
an information apparatus that extracts image information from said light captured by said second optical signal capturing apparatus, wherein
said output apparatus also provides said information as a function of said extracted image information.

3. The system of claim 2, wherein said information apparatus extracts said image information from said light captured by said second optical signal capturing apparatus based on positioning information.

4. The system of any one of the preceding claims, wherein said evaluation apparatus ascertains said orientation of said eye relative to said natural environment of said eye.

5. The system of any one of the preceding claims, wherein said projection apparatus projects said visible images into said eye in such a manner that said natural environment of said eye and said visible images are perceived as a unitary image.

6. The system of any one of the preceding claims, comprising a control desk (1412), wherein said first and second signal capturing apparatuses are part of a helmet system.

7. The system of any one of the preceding claims, comprising a position sensor, in particular a GPS receiver and/or an acceleration measurement apparatus.

8. The system of claim 7, comprising a database (1640) or a database connection that supplies orientation/navigation data.

9. The system of any one of the preceding claims, wherein said first and second signal capturing apparatuses are part of spectacles that constitute said first coordinate system.

10. The system of any one of the preceding claims, wherein said first signal capturing apparatus regularly carries out a signal capture with low processing demands and intermittently carries out a signal capture requiring intensive processing.

## Revendications

1. Système pour la mise à disposition d'informations, comprenant
un premier dispositif de détection de signaux optique (551) se trouvant dans un premier système de coordonnées, pour la détection de lumière qui a été renvoyée ou dégagée par un oeil ;
un second dispositif de détection de signaux optique (1061L, 1061R) se trouvant dans le premier système de coordonnées, pour la détection de lumière provenant de l'environnement naturel de l'oeil sans détecter de lumière qui a été réfléchie par l'oeil ;
un dispositif d'évaluation qui détermine le sens du regard de l'oeil par rapport au premier système de coordonnées à partir de la lumière détectée par le premier dispositif de détection de signaux optique, détermine la position et l'orientation du premier système de coordonnées à partir de la lumière détectée par le second dispositif de détection de signaux optique et établit ainsi une corrélation entre l'oeil et l'environnement ;
un dispositif de sortie qui met à disposition une information à l'aide de la corrélation entre l'oeil et l'environnement, dans lequel
le dispositif de sortie comprend un dispositif de projection (553) qui projette l'information dans l'oeil sous la forme d'images visibles, et
la lumière détectée par le second dispositif de détection de signaux optique est utilisée pour projeter les images visibles dans l'oeil de telle sorte que les images visibles semblent être immobiles lors d'un mouvement de l'oeil.

2. Système selon la revendication 1, comprenant
un dispositif d'information qui obtient des informations d'images à partir de la lumière détectée par le second dispositif de détection de signaux optique, dans lequel
le dispositif de sortie met à disposition les informations également en fonction des informations d'images obtenues.

3. Système selon la revendication 2, dans lequel le dispositif d'information obtient les informations d'images à partir de la lumière détectée par le second dispositif de détection de signaux optique à l'aide d'informations de localisation.

4. Système selon l'une quelconque des revendications précédentes, dans lequel le dispositif d'évaluation détermine l'orientation de l'oeil par rapport à l'environnement naturel de l'oeil.

5. Système selon l'une quelconque des revendications précédentes, dans lequel le dispositif de projection projette les images visibles dans l'oeil de telle sorte que l'environnement naturel de l'oeil et les images visibles sont perçus comme une image uniforme.

6. Système selon l'une quelconque des revendications précédentes, comprenant un pupitre de commande (1412), dans lequel les premier et second dispositifs de détection de signaux font partie d'un système de casque.

7. Système selon l'une quelconque des revendications précédentes, comprenant un capteur de position, notamment un récepteur GPS et/ou un dispositif de mesure de l'accélération.

8. Système selon la revendication 7, comprenant une base de données (1640) ou une liaison à une base de données qui fournit des données d'orientation.

9. Système selon l'une quelconque des revendications précédentes, dans lequel les premier et second dispositifs de détection de signaux optiques font partie d'une lunette qui représente le premier système de coordonnées.

10. Système selon l'une quelconque des revendications précédentes, dans lequel le premier dispositif de détection de signaux réalise régulièrement une détection de signaux pauvre en traitement et de manière intermittente une détection de signaux au traitement intensif.
